(19) Europäisches Patentamt
European Patent Office
Office européen des brevets

(11) Publication number: **0 024 172**
**B1**

(12) **EUROPEAN PATENT SPECIFICATION**

(45) Date of publication of patent specification: **11.05.83**

(21) Application number: **80302716.8**

(22) Date of filing: **07.08.80**

(51) Int. Cl.³: **C 07 D 233/70,**
C 07 D 401/12,
C 07 D 403/12,
C 07 D 405/12,
C 07 D 409/12
//A61K31/415, A61K31/44,
A61K31/455

(54) **Antitumor and immunosuppressive 4-carbamoylimidazolium-5-olate derivatives, pharmaceutical composition and production thereof.**

(30) Priority: **08.08.79 JP 101684/79**
**20.09.79 JP 121783/79**
**04.04.80 JP 45042/80**

(43) Date of publication of application:
**25.02.81 Bulletin 81/8**

(45) Publication of the grant of the patent:
**11.05.83 Bulletin 83/19**

(84) Designated Contracting States:
**AT BE CH DE FR GB IT LI NL SE**

(56) References cited:
**DE - A - 2 740 281**
**DE - A - 2 838 892**
**JP - A - 53 005 162**
**JP - A - 53 053 652**
**US - A - 4 140 788**

**The file contains technical information submitted after the application was filed and not included in this specification**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Atsumi, Toshio**
**3-45 Seiwadai Nishi 2-chome**
**Kawanishi (JP)**
Inventor: **Seta, Akinori**
**11-8-505 Sone Higashimachi 2-chome**
**Toyonaka (JP)**
Inventor: **Sanjiki, Tetsutaro**
**7-6 Funakicho**
**Ibaraki (JP)**
Inventor: **Kiyohara, Takao**
**14-7 Mefu 2-chome**
**Takarazuka (JP)**

(74) Representative: **Day, Jeremy John et al,**
**REDDIE & GROSE 16 Theobalds Road**
**London, WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 024 172 B1

Antitumor and immunosuppressive 4-carbamoylimidazolium-5-olate derivatives, pharmaceutical composition and production thereof

The present invention relates to 4-carbamoylimidazolium-5-olate derivatives useful as antitumor agents and/or immunosuppressants, to their preparation, and to pharmaceutical compositions containing them.

The use of 4-carbamoyl-5-hydroxyimidazole in the treatment of cancer, rheumatism and nephritis is described in German Offenlegungsschrift No. 2,740,281.

German Offenlegungsschrift No. 2,838,892 discloses certain 2-substituted-4-carbamoyl-imidazolium-5-olates and U.S. Patent No. 4,140,788 compounds having the formula

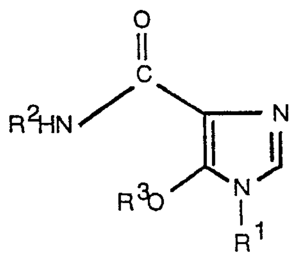

in which $R^1$ is a hydrogen atom or a lower alkyl, lower alkenyl or aralkyl group, $R^2$ is a hydrogen atom or a lower alkyl, lower alkenyl, aralkyl or 1-adamantyl group and $R^3$ is a hydrogen atom or a lower alkyl or aralkyl group and in which one of $R^1$, $R^2$ and $R^3$ is not a hydrogen atom when the other two are hydrogen atoms.

The compounds of this U.S. Patent and those of German Offenlegungsschrift No. 2,838,892 can be useful as anti-cancer agents.

It is known from Japanese Patent Publications (Kokai) Nos. 53—5162, 53—53652 that antitumor and immuno-suppressive activity is shown by compounds of the following formula (IV):

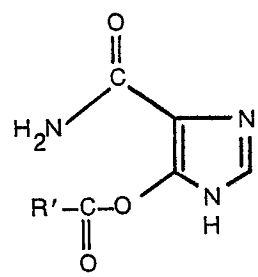

(IV)

wherein

R′ is an alkyl group, an adamantyl group or a phenyl group which may be substituted with an alkyl group, an alkoxy group, an alkylthio group, a halogen atom, a nitro group, a cyano group, a methylenedioxy group or an acetamido group.

The compounds of formula (IV) are, however, practically insoluble in aqueous media, so that their usefulness in therapy is restricted.

The present invention provides compounds of the following formula (I), and non-toxic salts thereof:

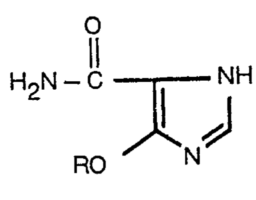

(I)

wherein

R is a heteroaroyl group having a 5- or 6-membered ring containing a nitrogen, sulfur or oxygen atom which may be substituted with a halogen atom, a $C_1$ to $C_6$ alkyl group, a $C_7$ or $C_8$ aralkyl group, a $C_1$ to $C_6$ alkanoyl group, or an aroyl group which may itself be substituted with a $C_1$ to $C_6$ alkyl group, a $C_1$ to $C_6$ alkoxy group or a halogen atom; a $C_1$ to $C_{18}$ alkyl sulfonyl group; a benzenesulfonyl group which may be substituted with a $C_1$ to $C_6$ alkyl group, a $C_1$ to $C_6$ alkoxy group, a nitro group or a halogen atom; or a cycloalkanoyl group having a 3- to 8-membered ring which may be substituted with a $C_1$ to $C_6$ alkyl group, a $C_2$ to $C_6$ alkenyl group, a halogen atom, a $C_2$ to $C_6$ halogenoalkenyl group (e.g. 2,2-dichlorovinyl), a phenyl group, a halogeno-phenyl group (e.g. *p*-chlorophenyl), a phenyl group substituted with a $C_1$ to $C_6$ alkyl or $C_1$ to $C_6$ alkoxy group (e.g. *p*-methoxyphenyl), a formyl group, a

hydroxy group, an amino group, a carboxyl group, an aminomethyl group or an oxo group.

Suitable heteroaroyl groups include furancarbonyl, thiophenecarbonyl, pyrrolecarbonyl, pyridinecarbonyl, pyridinecarbonyl N-oxide, pyrazinecarbonyl, imidazolecarbonyl, pyrazolecarbonyl, and thiazolecarbonyl.

Suitable substituted heteroaroyl groups include pyridinecarbonyl substituted with a lower alkyl group (e.g. methyl, n-butyl), thiophenecarbonyl substituted with a lower alkyl group (e.g. methyl), pyrrolecarbonyl substituted with a lower alkyl group (e.g. methyl), furancarbonyl substituted with an aralkyl group (e.g. benzyl), furancarbonyl substituted with an acyl group (e.g. acetyl), pyridinecárbonyl substituted with a halogen atom (e.g. chlorine, fluorine), and pyridinecarbonyl substituted with an acyl group (e.g. benzoyl).

Suitable cycloalkanoyl groups include cyclopropanecarbonyl, cyclobutanecarbonyl, cyclopentanecarbonyl, cyclohexanecarbonyl, cycloheptanecarbonyl, and cyclooctanecarbonyl.

The term "lower alkyl" herein means a straight or branched alkyl having 1 to 6 carbon atoms (e.g. methyl, ethyl, n-propyl, isopropyl, n-butyl, n-hexyl);

The term "lower alkoxy" herein means a straight or branched alkoxy having 1 to 6 carbon atoms (e.g. methoxy, ethoxy, n-propoxy, n-butoxy, isobutoxy, t-butoxy, n-hexyloxy);

The term "halogen" herein includes fluorine, chlorine, bromine and iodine;

The term "aralkyl" therein means an aralkyl having 7 to 8 carbon atoms such as benzyl, $\alpha$-methyl benzyl, phenethyl and the like;

The term "acyl" herein means lower alkanoyl having 1 to 6 carbon atoms (e.g. acetyl, propionyl) or aroyl (e.g. benzoyl) which may be substituted with a lower alkyl group, a lower alkoxy group or a halogen atom as defined above;

$C_2$ to $C_6$ alkenyl groups can have a straight or branched chain (e.g. vinyl, allyl, propenyl, hexenyl), as can the $C_1$ to $C_{18}$ alkyl groups (e.g. methyl, ethyl, n-propyl, isopropyl, n-butyl, isobutyl, t-butyl, pentyl, hexyl, heptyl, octyl, nonyl, decyl, hexadecyl, octadecyl).

The non-toxic salts of the compounds (I) of the present invention having a nitrogen atom in R may be formed with organic or inorganic acids such as hydrochloric acid, sulfuric acid, nitric acid, phospheric acid, p-toluenesulfonic acid, benzenesulfonic acid, methanesulfonic acid, tartaric acid, malic acid, lactic acid, maleic acid, or fumaric acid.

Compounds of the formula (I) can be prepared by reacting 4-carbamoylimidazolium-5-olate (II)

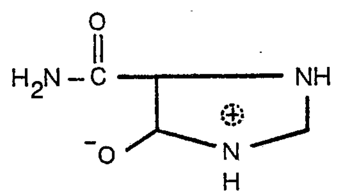

(II)

or a reactive derivative thereof with a reactive derivative of carboxylic acids or sulfonic acids of the formula (III):

$$R\text{—}OH \qquad (III)$$

wherein

R is as defined above.

Examples of preferred reactive derivatives of carboxylic acids or sulfonic acids of the formula (III) are carboxylic or sulfonic acid halides (e.g. chlorides, bromides, iodides, fluorides), carboxylic acid anhydrides, mixed anhydrides (e.g. mixed anhydrides with ethyl chloroformate, isobutyl chloroformate and the like), activated esters (e.g. p-nitrophenyl ester, ester with N-hydroxysuccinimide), imidazolide (e.g. prepared by reacting N,N'-carbonyldiimidazole with carboxylic acids), activated intermediates prepared by reacting carboxylic or sulfonic acids with reaction products obtained from N,N-dimethylformamide and oxalyl chloride (or phosgene or thionyl chloride or phosphorus pentachloride) and the like.

Examples of preferred reactive derivatives of 4-carbamoylimidazolium-5-olate of the formula (II) are trimethylsilyl derivatives, trialkyltin derivatives, mercury salts, silver salts and the like.

Typical examples of preferred solvents which may be used in this reaction are methylene chloride, chloroform, pyridine, diethyl ether, tetrahydrofuran, dioxane, benzene, toluene, methanol, ethanol, N,N-dimethylformamide, formamide, N,N-dimethylacetamide, acetonitrile, nitromethane, acetone, and ethyl acetate.

The reaction can generally be effected at a reaction temperature from —78° to 100°C., preferably from —60° to 60°C.

The reaction of 4-carbamoylimidazolium-5-olate with carboxylic or sulfonic acid halides can usually be carried out in an inert polar solvent or a mixture of water and inert organic solvent preferably in the presence of an inorganic or organic base at a temperature from —10° to 60°C. using one to two

mole equivalent of acid halide.

Typical examples of said inert polar solvent are tetrahydrofuran, dioxane, pyridine, N,N-dimethylformamide, formamide, N,N-dimethylacetamide and dimethylsulfoxide. Typical examples of said inert organic solvents are tetrahydrofuran, dioxane, diethyl ether, chlorform, dichloromethane, dichloroethane, benzene, toluene, and xylene. Examples of preferred inorganic bases are sodium hydroxide, sodium carbonate, sodium bicarbonate, potassium carbonate or bicarbonate and potassium hydroxide. Examples of preferred organic bases are pyridine, triethylamine and N,N-dimethylaniline.

The reaction of 4-carbamoylimidazolium-5-olate with activated intermediates prepared by reacting carboxylic or sulfonic acids with reaction products obtained from N,N-dimethylformamide and oxalyl chloride (or phosgene or thionyl chloride or phosphorus pentachloride) can usually be carried out in an organic solvent (e.g. acetonitrile, pyridine, N,N-dimethylformamide, N,N-dimethylacetamide, or chloroform) at a temperature from −78° to 80°C.

Compounds of formula (I) can also be prepared by reacting a silylated derivative of 4-carbamoylimidazolium-5-olate with reactive derivatives of carboxylic or sulfonic acids (e.g. acid halides) at a temperature from −78° to 50°C. in an inert organic solvent (e.g. dimethylformamide, tetrahydrofuran, dioxane, diethylether, benzene, or toluene).

The silylated derivative of 4-carbamoylimidazolium-5-olate are known and can be prepared by known methods (Hayashi, et al. Japanese Patent Publication (Kokai) No. 50—121276). When a compound of formula (I) is formed in the reaction mixture as a silylated derivative, the free compound can be obtained by a desilylating reaction with a desilylating reagent (e.g. acetic acid or methanol).

When an acid halide is used as the reactive derivative of acid (III), eliminated halide can be neutralised by an organic based (e.g. triethylamine or pyridine).

Compound of formula (I) where R is a cycloalkanoyl group substituted with an amino, hydroxy or carboxyl group can be prepared by the said acylation methods after protecting the amino, hydroxy or carboxyl group with a protective group (e.g. benzyl, benzyloxycarbonyl, t-butoxycarbonyl or the like), which is removed afterwards.

The compounds of formula (I) can be isolated and purified by known purification methods (e.g. recrystallization, column chromatography).

The imidazole derivatives of the present invention may exist in a mixture of the two tautomers as follows:

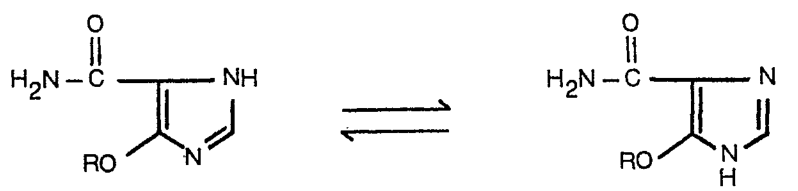

both of which are within the scope of the present invention.

Compounds of the present invention have shown antitumor activities against Sarcoma 180, Lewis lung carcinoma, Ehrlich carcinoma, and P—388 leukemia.

Compounds of formula (I) are useful as antitumor agents and can exhibit inhibitory effects against tumor and prolong life span.

The antitumor activities of Compounds of the present invention were estimated according to the methods described in "Cancer chemotherapy reports" Part 3, Vol. 3, (No. 2) p. 13 (1972). The results are given in the following Table 1.

Table 1

Antitumor effect on mouse experimental tumors

| Compound | Dose (mg/kg) Route i.p. | Schedule | Inhibition Ratio (%) Lewis lung carcinoma (solid) |
|---|---|---|---|
| 5-Carbamoyl-1H-imidazole-4-yl furan-2'-carboxylate | 100 | 5q2d | 48.4 |
| 5-Carbamoyl-1H-imidazole-4-yl thiophene-2'-carboxylate | 100 | 5q2d | 62.6 |
| 5-Carbamoyl-1H-imidazole-4-yl isonicotinate | 100 | 5q2d | 41.6 |
| 5-Carbamoyl-1H-imidazole-4-yl N-methylpyrrole-2'-carboxylate | 89.25 | 9qd | 57.3 |
| 5-Carbamoyl-1H-imidazole-4-yl furan-3'-carboxylate | 50 | 9qd | 34 |
| 5-Carbamoyl-1H-imidazole-4-yl benzenesulfonate | 100 | 9qd | 44.1 |
| 5-Carbamoyl-1H-imidazole-4-yl 4'-fluorobenzenesulfonate | 50 | 9qd | 48.2 |
| 5-Carbamoyl-1H-imidazole-4-yl 1'-methylcyclohexane-1'-carboxylate | 100 | 5q2d | 60.1 |
| 5-Carbamoyl-1H-imidazole-4-yl cyclobutane-1'-carboxylate | 100 | 5q2d | 33 |

$BDF_1$ male mice, 5 weeks old, weighing between 18 and 22 grams were used. Each test group was composed of 6 to 7 mice. Two million cells of Lewis Lung Carcinoma were injected in the hind leg. The drug was administered intraperitoneally at days 1, 3, 5, 7 and 9 (i.e. 5q2d) or on each of days 1 to 9 (i.e. 9qd).

After killing the mice at day 13, tumors were removed and weighed. The tumor inhibitory ratio was calculated according to the following formula.

$$\text{Inhibition ratio} = \left(1 - \frac{\text{the mean tumor weights of treated group}}{\text{the mean tumor weights of control group}}\right) \times 100$$

Compounds of the present invention can possess immunosuppressive activity as well as antitumor activity.

Compounds (I) of the present invention can have low toxicity, not showing toxic symptoms even when over 1000 mg/kg is orally administered to a mouse. Moreover, they may not exhibit an influence on the decrease of peripheral leucocytes, which is one of the most serious side effects of immunosuppressants.

Compounds of the present invention can be administered orally or parenterally to a warm-blood animal at a daily dose of 2—200 mg/kg as an antitumor agent, and 1—100 mg/kg as an immunosuppressant agent, in conventional dosage unit form.

The compounds of the present invention may be made up alone or together with a conventional

pharmaceutical carrier or diluent as a conventional solid or liquid pharmaceutical preparation (e.g. powders, granules, tablets, capsules, suspensions, emulsions, solutions) using methods conventional in the pharmaceutical field. For example, tablets or capsules may contain 50—500 mg of compounds (I).

The compounds (I) of the present invention can in particular be used for injection and as drops, being water soluble.

The following Examples are giving to illustrate the present invention more precisely but it is not intended to limit the present invention thereto.

Example 1

To a suspension of 0.636 g. of 4-carbamoylimidazolium-5-olate in 15 ml of dry pyridine was added dropwise 0.848 g. of 2-furoyl chloride at a temperature below 5°C. in an $N_2$ atmosphere. After stirring for two hours at 41—43°C., the reaction mixture was cooled to room temperature and 0.658 g. of triethylamine was added. Then the reaction mixture was concentrated under reduced pressure. To the residue was added chloroform and the separated crystals were filtered off, washed with chloroform, toluene and diethyl ether, and dried to give 0.789 g. of 5-carbamoyl-1H-imidazole-4-yl furan-2'-carboxylate, m.p. 161—163°C. (dec.). The crude material was recrystallized from N,N-dimethylformamide and diisopropyl ether after washing with a small quantity of water.
m.p.: 186.5°C. (dec.)
$\nu_{max}^{nujol}$ ($cm^{-1}$): 3450, 3320, 3125, 1760, 1655, 1600, 1485, 1280, 1160

Elemental analysis:

| | C (%) | H (%) | N (%) |
|---|---|---|---|
| Calculated for $C_9H_7O_4N_3$ | 48.89 | 3.19 | 19.00 |
| Found | 48.8 | 3.4 | 18.8 |

Example 2

Following a procedure similar to that of Example 1 but using 0.636 g. of 4-carbamoylimidazolium-5-olate and 0.880 g. of 2-thiophenecarbonyl chloride there was obtained 0.993 g. of 5-carbamoyl-1H-imidazole-4-yl thiophene-2'-carboxylate. m.p.: 198.5—199°C.

The crude material was recrystallized from N,N-dimethylformamide and water. m.p.: 199—199.5°C.
$\nu_{max}^{nujol}$ ($cm^{-1}$): 3475, 3450, 3200—3100, 1730, 1670, 1605, 1460, 1380, 1265, 1120

Elemental analysis:

| | C (%) | H (%) | N (%) | S (%) |
|---|---|---|---|---|
| Calculated for $C_9H_7O_3SN_3$ | 45.57 | 2.97 | 17.71 | 13.52 |
| Found | 45.36 | 2.77 | 17.94 | 13.14 |

Example 3

Following a procedure similar to that of Example 1 but using 0.636 g. of 4-carbamoyl imidazolium-5-olate, 1.157 g. of nicotinoyl chloride hydrochloride and 1.315 g. of triethylamine there was obtained 0.988 g. of 5-carbamoyl-1H-imidazole-4-yl nicotinate. m.p.: 183.5°C. (charred)
$\nu_{max}^{nujol}$ ($cm^{-1}$): 3440, 3150, 1740, 1670, 1605, 1590, 1460, 1420, 1275, 1130, 1015, 725

Elemental analysis:

| | C (%) | H (%) | N (%) |
|---|---|---|---|
| Calculated for $C_{10}H_8N_4O_3 \cdot 0.05H_2O$ | 51.52 | 3.50 | 24.03 |
| Found | 51.25 | 3.31 | 24.04 |

Example 4

Following a procedure similar to that of Example 3 but using 0.636 g. of 4-carbamoyl imidazolium-5-olate and 1.068 g. of isonicotinoyl chloride hydrochloride there was obtained 1.16 g. of 5-carbamoyl-1H-imidazol-4-yl isonicotinate. m.p.: 186°C. (charred)

The crude material was recrystallized from dimethylsulfoxide and water. m.p.: 192.5°C. (charred)
$\nu_{max}^{nujol}$ ($cm^{-1}$): 3440, 3400—3000, 1760, 1650, 1590, 1420, 1250, 1100, 1045

Elemental analysis:

| | C (%) | H (%) | N (%) |
|---|---|---|---|
| Calculated for $C_{10}H_8N_4O_3 \cdot 0.3H_2O$ | 50.55 | 3.65 | 23.58 |
| Found | 50.8 | 3.6 | 23.5 |

Example 5

To a suspension of 0.636 g. of 4-carbamoylimidazolium-5-olate in 15 ml. of dry pyridine was slowly added 1.068 g. of isonicotinoyl chloride hydrochloride at a temperature below 5°C. in an $N_2$ atmosphere. After stirring for an hour at 41—43°C., the reaction mixture was cooled to room

temperature. The separated crystals were filtered off, washed with pyridine, chloroform, ethyl acetate and diethyl ether, and dried to give 1.194 g of 5-carbamoyl-1H-imidazole-4-yl isonicotinate hydrochloride.
$\nu_{max}^{nujol}$ ($cm^{-1}$): 3340, 3150—3100, 1755, 1660, 1605, 1585, 1460, 1380, 1250, 1050, 750

Example 6

To a stirred solution of 12.5 ml. of N,N-dimethylformamide in 7.5 ml. of acetonitrile was added slowly 0.9 ml. of oxalyl chloride in 10 ml. of acetonitrile at −20°C. and stirring was continued for twenty minutes at −15°C. To the reaction mixture was slowly added 1.50 g. of N-methylpyrrole-2-carboxylic acid at −15°C. and stirring was continued for forty five minutes at 0°C. To the reaction mixture was slowly added 1.272 g. of 4-carbamoylimidazolium-5-olate and 10 ml of pyridine at −20C. and stirring was continued for ninety minutes at room temperature. After 200 ml. of chloroform was added to the reaction mixture, separated precipitate was filtered off and the filtrate was concentrated under reduced pressure.

To the residue were added 30 ml. of pyridine and 3.8 ml. of triethylamine and separated precipitate was filtered off and the filtrate was concentrated under reduced pressure to give 1.17 g. of 5-carbamoyl-1H-imidazole-4-yl N-methylpyrrole-2′-carboxylate, m.p. 174°C. (dec.) which was recrystallized from ethanol. m.p.: 189.5°C. (dec.).
$\nu_{max}^{nujol}$ ($cm^{-1}$): 3470, 3430, 3350, 3300, 3250, 1705, 1660, 1640, 1605, 1595, 1305, 1280, 1235, 1215, 1160, 1105, 1055, 1040, 990, 880

Elemental analysis:

| | C (%) | H (%) | N (%) |
|---|---|---|---|
| Calculated for $C_{10}H_{10}N_4O_3$ | 51.27 | 4.30 | 23.92 |
| Found | 50.99 | 4.30 | 23.77 |

Example 7

To a stirred solution of 12.5 ml. of N,N-dimethylformamide in 7.5 ml. of acetonitrile was added slowly a solution of 0.9 ml. of oxalyl chloride in 10 ml. of acetonitrile at −20°C. and the reaction temperature was raised to 20°C. from −15°C. over a period of 30 minutes. To the reaction mixture was slowly added 1.77 g of furan-3-carboxylic acid at −25°C. and stirring was continued for 16 hours at room temperature. To the reaction mixture were slowly added 1.272 g. of 4-carbamoyl imidazolium-5-olate and 5 ml. of pyridine at 0°C. and stirring was continued for four hours at room temperature. After 3.8 ml. of triethylamine was added to the reaction mixture, separated precipitate was filtered off.

To the filtrate was added 170 ml. of chloroform, and the separated crystals were filtered off and dried to give 1.55 g. of 5-carbamoyl-1H-imidazole-4-yl furan-3′-carboxylate. m.p.: 173°C. (dec.)

The crude material was recrystallized from methanol and water. m.p.: 198°C. (dec.)
$\nu_{max}^{nujol}$ ($cm^{-1}$): 3480, 3460, 1750, 1680, 1610, 1570, 1335, 1315, 1160, 1135, 1070

Elemental analysis:

| | C (%) | H (%) | N (%) |
|---|---|---|---|
| Calculated for $C_9H_7O_4N_3$ | 48.87 | 3.19 | 19.00 |
| Found | 48.5 | 3.2 | 18.8 |

Example 8

To a stirred suspension of 0.636 g. of 4-carbamoylimidazolium-5-olate in 15 ml. of dry pyridine was added 0.883 g. of 5-methyl-2-thiophenecarbamoyl chloride at a temperature below 5°C. in an $N_2$ atmosphere. After stirring for two hours at 41—43°C., the reaction mixture was cooled to room temperature and the separated crystals were filtered off and dried to give 1.449 g. of white crystals.

0.815 g. of crude material was recrystallized from N,N-dimethylformamide and water to give 0.513 g. of 5-carbamoyl-1H-imidazole-4-yl 5′-methylthiophene-2′-carboxylate. m.p.: 189.5°C. (charred)
$\nu_{max}^{nujol}$ ($cm^{-1}$): 3460, 1730, 1660, 1605

Elemental analysis:

| | C (%) | H (%) | N (%) | S (%) |
|---|---|---|---|---|
| Calculated for $C_{10}H_9N_3SO_3$ | 47.80 | 3.61 | 16.72 | 12.76 |
| Found | 47.52 | 3.59 | 16.50 | 12.54 |

Example 9

To a suspension of 0.636 g. of 4-carbamoylimidazolium-5-olate in 15 ml. of dry pyridine was added 0.80 ml. of benzenesulfonyl chloride at a temperature below 5°C. and stirring was continued for an hour at a temperature below 5°C. and for 20 hours at room temperature. To the reaction mixture was added 0.9 ml. of triethylamine and separated salts were filtered off. The filtrate was concentrated under reduced pressure. To the residue was added chloroform and the separated crystals were filtered

off, washed with chloroform and diethyl ether and dried to give 1.22 g. of 5-carbamoyl-1H-imidazole-4-yl benzenesulfonate.

0.530 g. of crude material was recrystallized from N,N-dimethylformamide and water. Amount: 0.400 g. m.p.: 147.5—148.0°C.

$\nu_{max}^{nujol}$ (cm$^{-1}$): 3470, 1680, 1610, 1580, 1420, 1190, 1090, 950, 820

Elemental analysis:

| | C (%) | H (%) | N (%) | S (%) |
|---|---|---|---|---|
| Calculated for $C_{10}H_9N_3O_4S$ | 44.93 | 3.39 | 15.72 | 12.00 |
| Found | 45.4 | 3.6 | 15.6 | 11.41 |

Example 10

Following a procedure similar to that of Example 9 but using 0.636 g. of 4-carbamoylimidazolium-5-olate and 1.240 g. of p-toluenesulfonyl chloride there was obtained 1.24 g. of 5-carbamoyl-1H-imidazole-4-yl 4′-methylbenzenesulfonate.

0.510 g. of crude material was recrystallized from N,N-dimethylformamide and water. Amount: 0.440 g. m.p.: 153°C. (dec.)

$\nu_{max}^{nujol}$ (cm$^{-1}$): 3450, 3150, 1660, 1605, 1420, 1190, 1170, 1080, 940

Elemental analysis:

| | C (%) | H (%) | N (%) | S (%) |
|---|---|---|---|---|
| Calculated for $C_{11}H_{11}N_3O_4S$ | 46.79 | 3.94 | 14.89 | 11.36 |
| Found | 46.77 | 3.94 | 14.76 | 11.21 |

Example 11

Following a procedure similar to that of Example 9, but using 0.636 g. of 4-carbamoylimidazolium-5-olate and 1.372 g. of p-chlorobenzenesulfonyl chloride and stirring for 20 hours at room temperature and then for an hour at 45°C. there was obtained 0.700 g. of 5-carbamoyl-1H-imidazole-4-yl 4′-chlorobenzenesulfonate.

The crude material was recrystallized from N,N-dimethylformamide and water. Amount: 0.595 g. m.p.: 158°C. (dec.)

$\nu_{max}^{nujol}$ (cm$^{-1}$): 3470, 1660, 1610, 1570, 1420, 1190, 1170, 1090, 1010, 945

Elemental analysis:

| | C (%) | H (%) | N (%) | S (%) | Cl (%) |
|---|---|---|---|---|---|
| Calculated for $C_{10}H_8N_3O_4SCl$ | 39.80 | 2.67 | 13.93 | 10.63 | 11.75 |
| Found | 39.6 | 2.5 | 13.8 | 10.70 | 12.00 |

Example 12

Following a procedure similar to that of Example 11 but using 0.636 g. of 4-carbamoylimidazolium-5-olate and 1.44 g. of o-nitrobenzenesulfonyl chloride there was obtained 1.09 g. of 5-carbamoyl-1H-imidazole-4-yl 2′-nitrobenzenesulfonate.

0.520 g. of crude material was recrystallized from N,N-dimethylformamide and water. Amount: 0.470 g. m.p.: 171.5—174.0°C.

$\nu_{max}^{nujol}$ (cm$^{-1}$): 3470, 1670, 1610, 1550, 1420, 1195, 1100, 950

Elemental analysis:

| | C (%) | H (%) | N (%) | S (%) |
|---|---|---|---|---|
| Calculated for $C_{10}H_8N_4O_6S$ | 38.46 | 2.58 | 17.94 | 10.27 |
| Found | 38.3 | 2.5 | 18.0 | 10.17 |

Example 13

Following a procedure similar to that of Example 9 but using 0.636 g. of 4-carbamoylimidazolium-5-olate and 1.343 g. of p-methoxybenzenesulfonyl chloride there was obtained 1.31 g. of 5-carbamoyl-1H-imidazole-4-yl 4′-methoxybenzenesulfonate.

0.500 g. of crude material was recrystallized from methanol and water. Amount: 0.230 g. m.p.: 162—164°C.

$\nu_{max}^{nujol}$ (cm$^{-1}$): 3400, 1660, 1315, 1270, 1190, 1165, 1090, 1015, 945

Elemental analysis:

| | C (%) | H (%) | N (%) | S (%) |
|---|---|---|---|---|
| Calculated for $C_{11}H_{11}N_3O_5S$ | 44.43 | 3.73 | 14.14 | 10.79 |
| Found | 44.2 | 3.5 | 14.0 | 10.41 |

Example 14

Following a procedure similar to that of Example 9 but using 0.636 g. of 4-carbamoylimidazole-5-olate and 0.50 ml. of methansulfonyl chloride there was obtained 0.64 g. of 5-carbamoyl-1H-imidazole-4-yl methanesulfonate after recrystallization from N,N-dimethylformamide and chloroform. m.p.: 174—175°C.

$\nu_{max}^{nujol}$ ($cm^{-1}$): 3450, 3160, 1670, 1605, 1570, 1430, 1180, 1170, 1100, 980, 940

Elemental analysis:

| | C (%) | H (%) | N (%) | S (%) |
|---|---|---|---|---|
| Calculated for $C_5H_7N_3O_4S$ | 29.23 | 3.44 | 20.56 | 15.61 |
| Found | 29.06 | 3.40 | 20.45 | 15.31 |

Example 15

To a suspension of 1.272 g. of 4-carbamoylimidazolium-5-olate in 20 ml of dry pyridine was added 2.1 g. of p-fluorobenzenesulfonyl chloride at room temperature. Stirring was continued at room temperature overnight. To the reaction mixture was added 1.5 ml of triethylamine and separated salts were filtered off. The filtrate was concentrated under reduced pressure. To the residue was added diethyl ether and the separated crystals were filtered off and dried to give 3.04 g. of 5-carbamoyl-1H-imidazole-4-yl p-fluorobenzenesulfonate.

The crude material was recrystallized from methanol. m.p.: 159—160°C.

$\nu_{max}^{nujol}$ ($cm^{-1}$): 3510, 3405, 1680, 1600, 1580, 1500, 1380, 1365, 1200, 1160, 1105, 1095

Elemental analysis:

| | C (%) | H (%) | N (%) | S (%) |
|---|---|---|---|---|
| Calculated for $C_{10}H_8N_3O_4SF$ | 42.10 | 2.83 | 14.73 | 11.24 |
| Found | 41.95 | 2.72 | 14.4 | 10.98 |

Reference 1

A mixture of 76.26 g. of 4-carbamoylimidazolium-5-olate, 174.31 g. of 1,1,1,3,3,3-hexamethyldisilazane, 1.59 g. of ammonium sulfate and 500 g. of dry xylene was refluxed for 4 hours. The reaction mixture was concentrated under reduced pressure and a tris-trimethylsilylated derivative of 4-carbamoylimidazolium-5-olate was obtained. m.p.: 83—86.5°C.

Example 16

To a stirred solution of 1.718 g. of tris-trimethylsilylated derivative of 4-carbamoylimidazolium-5-olate in 15 ml. of dry tetrahydrofuran was added dropwise a solution of 0.733 g. of cyclohexanecarbonyl chloride in 5 ml. of dry tetrahydrofuran at −50°C. under an $N_2$ atmosphere. After stirring for half an hour at −50°C., 0.48 g. of dry methanol was added to the reaction mixture. After stirring for 30 minutes at −50°C., 0.51 g. of triethylamine was added.

The reaction mixture was heated up to room temperature and the separated crystals were filtered off, washed with tetrahydrofuran and chloroform and dried to give 0.737 g. of 5-carbamoyl-1H-imidazole-4-yl cyclohexane-1′-carboxylate. The filtrate of a tetrahydrofuran solution was concentrated and diethyl ether was added to the residue and separated crystals were filtered off, washed with diethyl ether and dried to give 0.251 g. of said product. Chloroform solution used for washing was concentrated and water was added to the residue and separated crystals were filtered off and dried to give 0.112 g. of said product.

The crude material was recrystallized from methanol, diethyl ether and n-hexane. m.p.: 172°C. (decomp.)

$\nu_{max}^{nujol}$ ($cm^{-1}$): 3490, 3100, 1780, 1675, 1615

Elemental analysis:

| | C (%) | H (%) | N (%) |
|---|---|---|---|
| Calculated for $C_{11}H_{15}N_3O_3 \cdot 0.15H_2O$ | 55.06 | 6.43 | 17.51 |
| Found | 55.03 | 6.59 | 17.73 |

Example 17

Following a procedure similar to that of Example 16 but using 0.523 g. of cyclopropanecarbonyl chloride there was obtained 0.805 g. of 5-carbamoyl-1H-imidazole-4-yl cyclopropane-1′-carboxylate. m.p.: 154°C.

Recrystallized was crude material from methanol and diethyl ether. m.p.: 156.5°C. (dec.)

$\nu_{max}^{nujol}$ ($cm^{-1}$): 3460, 3270, 1775, 1760, 1670, 1605

Elemental analysis:

| | C (%) | H (%) | N (%) |
|---|---|---|---|
| Calculated for $C_8H_9N_3O_3 \cdot 0.1H_2O$ | 48.78 | 4.71 | 21.33 |
| Found | 48.48 | 4.55 | 22.04 |

Example 18

To a stirred solution of 1.718 g. of tris-trimethylsilylated derivative of 4-carbamoylimidazolium-5-olate in 15 ml. of dry tetrahydrofuran was added dropwise a solution of 0.933 g. of 2,2-dimethyl-3-(2-methyl-1-propenyl)cyclopropanecarbonyl chloride in 5 ml. of dry tetrahydrofuran at —50°C. in an $N_2$ atmosphere. After stirring for half an hour at —50°C., 0.32 g. of dry methanol was added to the reaction mixture. After stirring for 20 minutes at —50°C., 0.51 g. of triethylamine was added.

The reaction mixture was heated up to room temperature and separated crystals were filtered off. The filtrate was concentrated under reduced pressure and diethyl ether was added to the residue and separated crystals were filtered off and dried to give 1.199 g. of 5-carbamoyl-1H-imidazole-4-yl 2′,2′-dimethyl-3′-(2-methyl-1-propenyl)cyclopropane-1′-carboxylate.

The crude material was recrystallized from dimethylsulfoxide and water. m.p.: 154.5°C.
$\nu_{max}^{nujol}$ (cm$^{-1}$): 3460, 3125, 1755, 1655, 1605

Elemental analysis:

| | C (%) | H (%) | N (%) |
|---|---|---|---|
| Calculated for $C_{14}H_{19}N_3O_3$ | 60.63 | 6.91 | 15.15 |
| Found | 60.3 | 6.8 | 15.1 |

Example 19

Following a procedure similar to that of Example 16 but using 1.138 g. of 2,2-dimethyl-3-(2,2-dichlorovinyl)cyclopropanecarbonyl chloride there was obtained 0.670 g. of 5-carbamoyl-1H-imidazole-4-yl 2′,2′-dimethyl-3′-(2,2-dichlorovinyl)cyclopropane-1′-carboxylate.

The crude material was recrystallized from dimethylsulfoxide and water. m.p.: 175—176°C.
$\nu_{max}^{nujol}$ (cm$^{-1}$): 3450, 1750, 1655, 1600

Elemental analysis:

| | C (%) | H (%) | N (%) | Cl (%) |
|---|---|---|---|---|
| Calculated for $C_{12}H_{13}N_3O_3Cl_2 \cdot 0.5H_2O$ | 44.05 | 4.31 | 12.84 | 21.67 |
| Found | 43.83 | 4.09 | 12.84 | 20.57 |

Example 20

Following a procedure similar to that of Example 16 but using 0.803 g. of 2,2,3,3-tetramethyl-cyclopropanecarbonyl chloride there was obtained 0.891 g. of 5-carbamoyl-1H-imidazole-4-yl 2′,2′,3′,3′-tetramethylcyclopropane-1′-carboxylate.

The crude material was recrystallized from methanol, diethyl ether and bexane. m.p.: 172°C. (dec.)
$\nu_{max}^{nujol}$ (cm$^{-1}$): 3460, 3160, 1760, 1660, 1600

Elemental analysis:

| | C (%) | H (%) | N (%) |
|---|---|---|---|
| Calculated for $C_{12}H_{17}N_3 \cdot 0.1H_2O$ | 56.95 | 6.85 | 16.60 |
| Found | 56.80 | 6.86 | 16.68 |

Example 21

To a stirred suspension of 0.636 g. of 4-carbamoylimidazolium-5-olate in 15 ml. of dry pyridine was added dropwise 1.61 g. of 1-(p-chlorophenyl)-1-cyclopropanecarbonyl chloride at a temperature below 5°C. in an $N_2$ atmosphere. After stirring for two hours at 41 to 43°C., the reaction mixture was cooled to room temperature and 0.83 g. of triethylamine was added. Then the reaction mixture was concentrated under reduced pressure. To the residue was added diethyl ether and the separated crystals were filtered off, washed with diethyl ether, water and diisopropyl ether and dried to give 1.176 g. of 5-carbamoyl-1H-imidazole-4-yl 1′-(p-chlorophenyl)cyclopropane-1′-carboxylate. m.p.: 190°C. (charred)

The crude material was recrystallized from N,N-dimethylformamide and water. m.p.: 190—191°.
$\nu_{max}^{nujol}$ (cm$^{-1}$): 3450, 3125, 1740, 1650, 1600

Elemental analysis:

| | C (%) | H (%) | N (%) | Cl (%) |
|---|---|---|---|---|
| Calculated for $C_{14}H_{12}N_3O_3Cl$ | 55.00 | 3.96 | 13.74 | 11.60 |
| Found | 54.8 | 4.1 | 13.8 | 11.30 |

Example 22

To a stirred suspension of 0.636 g. of 4-carbamoylimidazolium-5-olate in 15 ml. of dry pyridine was added dropwise 1.61 g. of 1-methylcyclohexanecarbonyl chloride at a temperature below 5°C. in an $N_2$ atmosphere. After stirring for 3.5 hours at 41—43°C., the reaction mixture was concentrated under reduced pressure. To the residue was added water and the separated crystals were filtered off, washed with diisopropyl ether and dried to give 1.06 g. of 5-carbamoyl-1H-imidazole-4-yl 1′-methylcyclohexane-1′-carboxylate, m.p. 197.5—198°C.

The crude material was recrystallized from ethyl acetate. m.p.: 202.5—203°C.

$\nu_{max}^{nujol}$ (cm$^{-1}$): 3460, 3440, 3320, 3180, 1760, 1660, 1610

Elemental analysis:

| | C (%) | H (%) | N (%) |
|---|---|---|---|
| Calculated for $C_{12}H_{17}N_3O_3$ | 57.36 | 6.82 | 16.72 |
| Found | 57.3 | 6.8 | 16.6 |

Example 23

To a stirred suspension of 2.358 g. of 4-carbamoylimidazolium-5-olate in 60 ml. of dry pyridine was added dropwise 2.2 g. of 1-methylcyclopropanecarbonyl chloride at a temperature below 5°C. in an $N_2$ atmosphere. After stirring for two hours at 41—43°C., 2.07 g. of triethylamine was added to the reaction mixture. Separated salts were filtered off and the filtrate was concentrated under reduced pressure. 2.89 g. of 5-carbamoyl-1H-imidazole-4-yl 1′-methylcyclopropane-1′-carboxylate was obtained. The crude material was purified by silica gel column chromatography. m.p.: 173.5—174°C.

$\nu_{max}^{nujol}$ (cm$^{-1}$): 3460, 3160, 1740, 1665, 1600

Elemental analysis:

| | C (%) | H (%) | N (%) |
|---|---|---|---|
| Calculated for $C_9H_{11}N_3O_3 \cdot 0.1H_2O$ | 51.23 | 5.35 | 19.91 |
| Found | 51.42 | 5.38 | 19.71 |

Example 24

Following a procedure similar to that of Example 23 but using 0.636 g. of 4-carbamoylimidazolium-5-olate, 15 ml. of dry pyridine, 1.03 g. of 2,2-dichloro-1-methylcyclopropanecarbonyl chloride and 0.612 g. of triethylamine there was obtained 1.123 g. of 5-carbamoyl-1H-imidazole-4-yl 2′,2′-dichloro-1′-methylcyclopropane-1′-carboxylate, m.p. 169—172°C. (charred).

$\nu_{max}^{nujol}$ (cm$^{-1}$): 3460, 3320, 3150, 1760, 1680, 1600

Elemental analysis:

| | C (%) | H (%) | N (%) | Cl (%) |
|---|---|---|---|---|
| Calculated for $C_9H_9N_3O_3Cl_2$ | 38.87 | 3.26 | 15.11 | 25.50 |
| Found | 38.96 | 3.31 | 15.02 | 25.47 |

Example 25

Following a procedure similar to that of Example 16 but using 0.593 g. of cyclobutanecarbonyl chloride there was obtained 0.752 g. of 5-carbamoyl-1H-imidazole-4-yl cyclobutane-1′-carboxylate.

The crude material was recrystallized from N,N-dimethylformamide, tetrahydrofuran, diethyl ether and hexane. m.p.: 160°C. (dec.)

$\nu_{max}^{nujol}$ (cm$^{-1}$): 3430, 3320, 3150, 1740, 1670, 1605

According to the present invention, there may also be obtained, for example, the following compounds:

5-carbamoyl-1H-imidazole-4-yl thiophene-3′-carboxylate,
5-carbamoyl-1H-imidazole-4-yl picolinate,
5-carbamoyl-1H-imidazole-4-yl picolinate N-oxide,
5-carbamoyl-1H-imidazole-4-yl nicotinate N-oxide,
5-carbamoyl-1H-imidazole-4-yl isonicotinate N-oxide,
5-carbamoyl-1H-imidazole-4-yl pyrazine-2′-carboxylate,
5-carbamoyl-1H-imidazole-4-yl 5′-benzylfuran-3′-carboxylate,
5-carbamoyl-1H-imidazole-4-yl 6′-chloronicotinate,
5-carbamoyl-1H-imidazole-4-yl 2′-chloronicotinate,
5-carbamoyl-1H-imidazole-4-yl 5′-acetylfuran-2′-carboxylate,
5-carbamoyl-1H-imidazole-4-yl ethanesulfonate,
5-carbamoyl-1H-imidazole-4-yl propane-1′-sulfonate,
5-carbamoyl-1H-imidazole-4-yl butane-1′-sulfonate,
5-carbamoyl-1H-imidazole-4-yl pentane-1′-sulfonate,
5-carbamoyl-1H-imidazole-4-yl hexane-1′-sulfonate,

5-carbamoyl-1H-imidazole-4-yl decane-1′-sulfonate,
5-carbamoyl-1H-imidazole-4-yl hexadecane-1′-sulfonate,
5-carbamoyl-1H-imidazole-4-yl octadecane-1′-sulfonate,
5-carbamoyl-1H-imidazole-4-yl cyclopentane-1′-carboxylate,
5-carbamoyl-1H-imidazole-4-yl cycloheptane-1′-carboxylate,
5-carbamoyl-1H-imidazole-4-yl cyclooctane-1′-carboxylate,
5-carbamoyl-1H-imidazole-4-yl 1′-(p-methoxyphenyl)cyclopentane-1′-carboxylate,
5-carbamoyl-1H-imidazole-4-yl 1′-phenylcyclopentane-1′-carboxylate,
5-carbamoyl-1H-imidazole-4-yl 1′-(p-tolyl)cyclopentane-1′-carboxylate,
5-carbamoyl-1H-imidazole-4-yl 3′-formylcyclohexane-1′-carboxylate,
5-carbamoyl-1H-imidazole-4-yl 1′-hydroxycyclohexane-1′-carboxylate,
5-carbamoyl-1H-imidazole-4-yl 1′-aminocyclopentane-1′-carboxylate,
5-carbamoyl-1H-imidazole-4-yl 2′-carboxycyclohexane-1′-carboxylate,
5-carbamoyl-1H-imidazole-4-yl 4′-aminomethylcyclohexane-1′-carboxylate,
5-carbamoyl-1H-imidazole-4-yl 3′-oxocyclopentane-1′-carboxylate,
5-carbamoyl-1H-imidazole-4-yl 1′-chloro-2′-oxocyclopentane-1′-carboxylate.

**Claims for the Contracting States: BE CH DE FR GB IT LI NL SE**

1. A compound of the formula, or a non-toxic salt thereof:

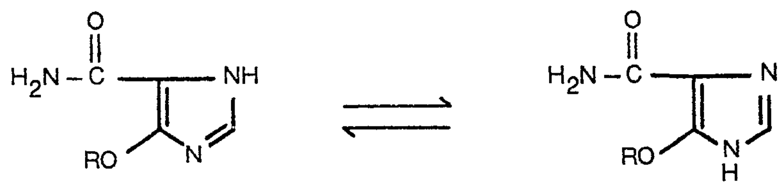

wherein

R is a heteroaroyl group having a 5- or 6-membered ring containing a nitrogen, sulfur or oxygen atom which may be substituted with a halogen atom, a $C_1$ to $C_6$ alkyl group, a $C_7$ or $C_8$ aralkyl group, a $C_1$ to $C_6$ alkanoyl group, or an aroyl group which may itself be substituted with a $C_1$ to $C_6$ alkyl group, a $C_1$ to $C_6$ alkoxy group or a halogen atom; a $C_1$ to $C_{18}$ alkyl sulfonyl group; a benzenesulfonyl group which may be substituted with a $C_1$ to $C_6$ alkyl group, a $C_1$ to $C_6$ alkoxy group, a nitro group or a halogen atom; or a cycloalkanoyl group having a 3- to 8-membered ring which may be substituted with a $C_1$ to $C_6$ alkyl group, a $C_2$ to $C_6$ alkenyl group, a halogen atom, a $C_2$ to $C_6$ halogeno-alkenyl group, a phenyl group, a halogenophenyl group, a phenyl group substituted with a $C_1$ to $C_6$ alkyl or $C_1$ to $C_6$ alkoxy group, a formyl group, a hydroxy group, an amino group, a carboxyl group, an aminomethyl group or an oxo group.

2. A compound according to claim 1 wherein R is 2-furancarbonyl, 2-thiophenecarbonyl, nicotinoyl, isonicotinoyl, isonicotinoyl hydrochloride, or 3-furancarbonyl.

3. A compound according to claim 1 wherein R is benzenesulfonyl, *p*-toluenesulfonyl, *p*-chlorobenzenesulfonyl, *o*-nitrobenzenesulfonyl, *p*-methoxybenzenesulfonyl, or *p*-fluorobenzenesulfonyl.

4. A compound according to claim 1 wherein R is methansulfonyl.

5. A compound according to claim 1 wherein R is 1-cyclohexanecarbonyl, 1-cyclopropanecarbonyl, or 1-cyclobutanecarbonyl.

6. A compound according to claim 1 wherein R is N-methylpyrrole-2-carbonyl or 5-methyl-2-thiophenecarbonyl.

7. A compound according to claim 1 wherein R is 2,2-dimethyl-3-(2-methyl-1-propenyl)-1-cyclopropanecarbonyl, 2,2-dimethyl-3-(2,2-dichlorovinyl)-1-cyclopropanecarbonyl, 2,2,3,3-tetramethyl-1-cyclopropanecarbonyl, 1-(*p*-chlorophenyl)-1-cyclopropanecarbonyl, 1-methyl-1-cyclohexanecarbonyl, 1-methyl-1-cyclopropanecarbonyl, or 2,2-dichloro-1-methyl-1-cyclopropanecarbonyl.

8. 5-carbamoyl-1H-imidazole-4-yl cyclopentane-1′-carboxylate,
5-carbamoyl-1H-imidazole-4-yl cycloheptane-1′-carboxylate,
5-carbamoyl-1H-imidazole-4-yl cyclooctane-1′-carboxylate,
5-carbamoyl-1H-imidazole-4-yl 1′-(p-methoxyphenyl)cyclopentane-1′-carboxylate,
5-carbamoyl-1H-imidazole-4-yl 1′-phenylcyclopentane-1′-carboxylate,
5-carbamoyl-1H-imidazole-4-yl 1′-(p-tolyl)cyclopentane-1′-carboxylate,
5-carbamoyl-1H-imidazole-4-yl 3′-formylcyclohexane-1′-carboxylate,
5-carbamoyl-1H-imidazole-4-yl 1′-hydroxycyclohexane-1′-carboxylate,
5-carbamoyl-1H-imidazole-4-yl 1′-aminocyclopentane-1′-carboxylate,
5-carbamoyl-1H-imidazole-4-yl 2′-carboxycyclohexane-1′-carboxylate,
5-carbamoyl-1H-imidazole-4-yl 4′-aminomethylcyclohexane-1′-carboxylate,
5-carbamoyl-1H-imidazole-4-yl 3′-oxocyclopentane-1′-carboxylate,
5-carbamoyl-1H-imidazole-4-yl 1′-chloro-2′-oxocyclopentane-1′-carboxylate,

5-carbamoyl-1H-imidazole-4-yl cyclohexane-1′-carboxylate,
5-carbamoyl-1H-imidazole-4-yl cyclopropane-1′-carboxylate,
5-carbamoyl-1H-imidazole-4-yl 2′,2′-dimethyl-3′-(2-methyl-1-propenyl)-cyclopropane-1′-carboxylate,
5-carbamoyl-1H-imidazole-4-yl 2′,2′-dimethyl-3′-(2,2-dichlorovinyl)cyclopropane-1′-carboxylate,
5-carbamoyl-1H-imidazole-4-yl 2′,2′,3′,3′-tetramethylcyclopropane-1′-carboxylate,
5-carbamoyl-1H-imidazole-4-yl 1′-(p-chlorophenyl)cyclopropane-1′-carboxylate,
5-carbamoyl-1H-imidazole-4-yl 1′-methylcyclohexane-1′-carboxylate,
5-carbamoyl-1H-imidazole-4-yl 1′-methylcyclopropane-1′-carboxylate,
5-carbamoyl-1H-imidazole-4-yl 2′,2′-dichloro-1′-methylcyclopropane-1′-carboxylate, or
5-carbamoyl-1H-imidazole-4-yl cyclobutane-1′-carboxylate.

9. 5-carbamoyl-1H-imidazole-4-yl picolinate,
5-carbamoyl-1H-imidazole-4-yl picolinate N-oxide,
5-carbamoyl-1H-imidazole-4-yl nicotinate N-oxide,
5-carbamoyl-1H-imidazole-4-yl isonicotinate,
5-carbamoyl-1H-imidazole-4-yl pyrazine-2′-carboxylate,
5-carbamoyl-1H-imidazole-4-yl 5′-benzylfuran-3′-carboxylate,
5-carbamoyl-1H-imidazole-4-yl 6′-chloronicotinate,
5-carbamoyl-1H-imidazole-4-yl 2′-chloronicotinate,
5-carbamoyl-1H-imidazole-4-yl 5′-acetylfuran-2′-carboxylate,
5-carbamoyl-1H-imidazole-4-yl nicotinate,
5-carbamoyl-1H-imidazol-4-yl isonicotinate,
5-carbamoyl-1H-imidazole-4-yl isonicotinate hydrochloride,
5-carbamoyl-1H-imidazole-4-yl N-methylpyrrole-2′-carboxylate,
5-carbamoyl-1H-imidazole-4-yl furan-2′-carboxylate,
5-carbamoyl-1H-imidazole-4-yl thiophene-2′-carboxylate,
5-carbamoyl-1H-imidazole-4-yl furan-3′-carboxylate,
5-carbamoyl-1H-imidazole-4-yl 5′-methylthiophene-2′-carboxylate, or
5-carbamoyl-1H-imidazole-4-yl thiophene-3′-carboxylate,

10. 5-carbamoyl-1H-imidazole-4-yl ethanesulfonate,
5-carbamoyl-1H-imidazole-4-yl propane-1′-sulfonate,
5-carbamoyl-1H-imidazole-4-yl butane-1′-sulfonate,
5-carbamoyl-1H-imidazole-4-yl pentane-1′-sulfonate,
5-carbamoyl-1H-imidazole-4-yl hexane-1′-sulfonate,
5-carbamoyl-1H-imidazole-4-yl decane-1′-sulfonate,
5-carbamoyl-1H-imidazole-4-yl hexadecane-1′-sulfonate,
5-carbamoyl-1H-imidazole-4-yl octadecane-1′-sulfonate, or
5-carbamoyl-1H-imidazole-4-yl methanesulfonate.

11. 5-carbamoyl-1H-imidazole-4-yl benzensulfonate,
5-carbamoyl-1H-imidazole-4-yl 4′-methylbenzenesulfonate,
5-carbamoyl-1H-imidazole-4-yl 4′-chlorobenzenesulfonate,
5-carbamoyl-1H-imidazole-4-yl 2′-nitrobenzenesulfonate,
5-carbamoyl-1H-imidazole-4-yl 4′-methoxybenzenesulfonate, or
5-carbamoyl-1H-imidazole-4-yl p-fluorobenzenesulfonate.

12. A process for producing a compound of the formula:

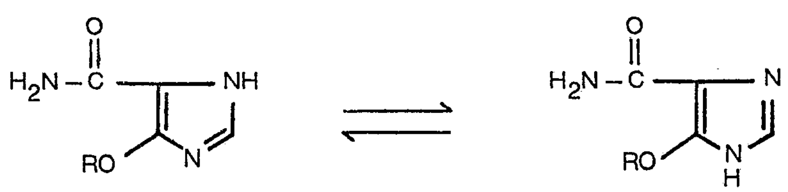

wherein

R is as defined in claim 1, which comprises reacting 4-carbamoylimidazolium-5-olate of the formula:

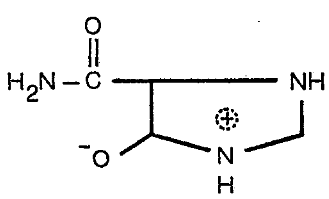

or a reactive derivative thereof with a reactive derivative of a carboxylic acid or sulfonic acid of the formula:

R—OH

wherein

R is as defined in claim 1.

13. An antitumor or immunosuppressant composition which comprises an effective amount of a compound according to any of claims 1 to 11 as an active ingredient and a pharmaceutically acceptable carrier or diluent.

**Claims for the Contracting State: AT**

1. A process for producing a compound of the formula:

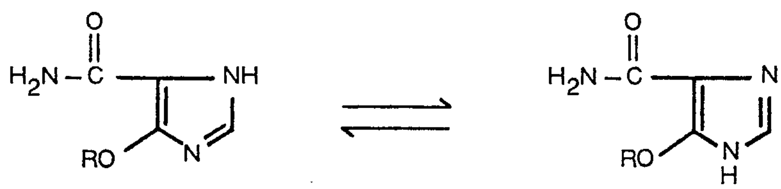

which comprises reacting 4-carbamoylimidazolium-5-olate of the formula:

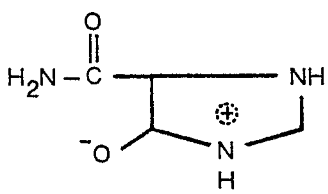

or a reactive derivative thereof with a reactive derivative of a carboxylic acid or sulfonic acid of the formula:

R—OH

wherein

R is a heteroaroyl group having a 5- or 6-membered ring containing a nitrogen, sulfur or oxygen atom which may be substituted with a halogen atom, a $C_1$ to $C_6$ alkyl group, a $C_7$ or $C_8$ aralkyl group, a $C_1$ to $C_6$ alkanoyl group, or an aroyl group which may itself be substituted with a $C_1$ to $C_6$ alkyl group, a $C_1$ to $C_6$ alkoxy group or a halogen atom; a $C_1$ to $C_{18}$ alkyl sulfonyl group; a benzenesulfonyl group which may be substituted with a $C_1$ to $C_6$ alkyl group, a $C_1$ to $C_6$ alkoxy group, a nitro group or a halogen atom; or a cycloalkanoyl group having a 3- to 8-membered ring which may be substituted with a $C_1$ to $C_6$ alkyl group, a $C_2$ to $C_6$ alkenyl group, a halogen atom, a $C_2$ to $C_6$ halogeno-alkenyl group, a phenyl group, a halogenophenyl group, a phenyl group substituted with a $C_1$ to $C_6$ alkyl or $C_1$ to $C_6$ alkoxy group, a formyl group, a hydroxy group, an amino group, a carboxyl group, an aminomethyl group or an oxo group.

2. A process according to claim 1 wherein R is 2-furancarbonyl, 2-thiophenecarbonyl, nicotinoyl, isonicotinoyl, isonicotinoyl hydrochloride, or 3-furancarbonyl.

3. A process according to claim 1 wherein R is benzenesulfonyl, *p*-toluenesulfonyl, *p*-chlorobenzenesulfonyl, *o*-nitrobenzenesulfonyl, *p*-methoxybenzenesulfonyl, or *p*-fluorobenzenesulfonyl.

4. A process according to claim 1 wherein R is methanesulfonyl.

5. A process according to claim 1 wherein R is 1-cyclohexanecarbonyl, 1-cyclopropanecarbonyl, or 1-cyclobutanecarbonyl.

6. A process according to claim 1 wherein R is N-methylpyrrole-2-carbonyl or 5-methyl-2-thiophenecarbonyl.

7. A process according to claim 1 wherein R is 2,2-dimethyl-3-(2-methyl-1-propenyl)-1-cyclopropanecarbonyl, 2,2-dimethyl-3-(2,2-dichlorovinyl)-1-cyclopropanecarbonyl, 2,2,3,3-tetramethyl-1-cyclopropanecarbonyl, 1-(p-chlorophenyl)-1-cyclopropanecarbonyl, 1-methyl-1-cyclohexanecarbonyl, 1-methyl-1-cyclopropanecarbonyl, or 2,2-dichloro-1-methyl-1-cyclopropanecarbonyl.

8. A process according to claim 1 for the production of
5-carbamoyl-1H-imidazole-4-yl cyclopentane-1′-carboxylate,
5-carbamoyl-1H-imidazole-4-yl cycloheptane-1′-carboxylate,
5-carbamoyl-1H-imidazole-4-yl cyclooctane-1′-carboxylate,
5-carbamoyl-1H-imidazole-4-yl 1′-(p-methoxyphenyl)cyclopentane-1′-carboxylate,

5-carbamoyl-1H-imidazole-4-yl 1′-phenylcyclopentane-1′-carboxylate,
5-carbamoyl-1H-imidazole-4-yl 1′-(p-tolyl)cyclopentane-1′-carboxylate,
5-carbamoyl-1H-imidazole-4-yl 3′-formylcyclohexane-1′-carboxylate,
5-carbamoyl-1H-imidazole-4-yl 1′-hydroxycyclohexane-1′-carboxylate,
5-carbamoyl-1H-imidazole-4-yl 1′-aminocyclopentane-1′-carboxylate,
5-carbamoyl-1H-imidazole-4-yl 2′-carboxycyclohexane-1′-carboxylate,
5-carbamoyl-1H-imidazole-4-yl 4′-aminomethylcyclohexane-1′-carboxylate,
5-carbamoyl-1H-imidazole-4-yl 3′-oxocyclopentane-1′-carboxylate,
5-carbamoyl-1H-imidazole-4-yl 1′-chloro-2′-oxocyclopentane-1′-carboxylate,
5-carbamoyl-1H-imidazole-4-yl cyclohexane-1′-carboxylate,
5-carbamoyl-1H-imidazole-4-yl cyclopropane-1′-carboxylate,
5-carbamoyl-1H-imidazole-4-yl 2′,2′-dimethyl-3′-(2-methyl-1-propenyl)-cyclopropane-1′-carboxylate,
5-carbamoyl-1H-imidazole-4-yl 2′,2′-dimethyl-3′-(2,2-dichlorovinylcyclopropane-1′-carboxylate,
5-carbamoyl-1H-imidazole-4-yl 2′,2′,3′,3′-tetramethylcyclopropane-1′-carboxylate,
5-carbamoyl-1H-imidazole-4-yl 1′-(p-chlorophenyl)cyclopropane-1′-carboxylate,
5-carbamoyl-1H-imidazole-4-yl 1′-methylcyclohexane-1′-carboxylate,
5-carbamoyl-1H-imidazole-4-yl 1′-methylcyclopropane-1′-carboxylate,
5-carbamoyl-1H-imidazole-4-yl 2′,2′-dichloro-1′-methylcyclopropane-1′-carboxylate, or
5-carbamoyl-1H-imidazole-4-yl cyclobutane-1′-carboxylate.

9. A process according to claim 1 for the production of
5-carbamoyl-1H-imidazole-4-yl picolinate,
5-carbamoyl-1H-imidazole-4-yl picolinate N-oxide,
5-carbamoyl-1H-imidazole-4-yl nicotinate N-oxide,
5-carbamoyl-1H-imidazole-4-yl isonicotinate N-oxide,
5-carbamoyl-1H-imidazole-4-yl pyrazine-2′-carboxylate,
5-carbamoyl-1H-imidazole-4-yl 5′-benzylfuran-3′-carboxylate,
5-carbamoyl-1H-imidazole-4-yl 6′-chloronicotinate,
5-carbamoyl-1H-imidazole-4-yl 2′-chloronicotinate,
5-carbamoyl-1H-imidazole-4-yl 5′-acetylfuran-2′-carboxylate,
5-carbamoyl-1H-imidazole-4-yl nicotinate,
5-carbamoyl-1H-imidazole-4-yl isonicotinate,
5-carbamoyl-1H-imidazole-4-yl isonicotinate hydrochloride,
5-carbamoyl-1H-imidazole-4-yl N-methylpyrrole-2′-carboxylate,
5-carbamoyl-1H-imidazole-4-yl furan-2′-carboxylate,
5-carbamoyl-1H-imidazole-4-yl thiophene-2′-carboxylate,
5-carbamoyl-1H-imidazole-4-yl furan-3′-carboxylate,
5-carbamoyl-1H-imidazole-4-yl 5′-methylthiophene-2′-carboxylate, or
5-carbamoyl-1H-imidazole-4-yl thiophene-3′-carboxylate,

10. A process according to claim 1 for the production of
5-carbamoyl-1H-imidazole-4-yl ethanesulfonate,
5-carbamoyl-1H-imidazole-4-yl propane-1′-sulfonate,
5-carbamoyl-1H-imidazole-4-yl butane-1′-sulfonate,
5-carbamoyl-1H-imidazole-4-yl pentane-1′-sulfonate,
5-carbamoyl-1H-imidazole-4-yl hexane-1′-sulfonate,
5-carbamoyl-1H-imidazole-4-yl decane-1′-sulfonate,

11. A process according to claim 1 for the production of
5-carbamoyl-1H-imidazole-4-yl hexadecane-1′-sulfonate,
5-carbamoyl-1H-imidazole-4-yl octadecane-1′-sulfonate, or
5-carbamoyl-1H-imidazole-4-yl methanesulfonate.
5-carbamoyl-1H-imidazole-4-yl benzenesulfonate,
5-carbamoyl-1H-imidazole-4-yl 4′-methylbenzenesulfonate,
5-carbamoyl-1H-imidazole-4-yl 4′-chlorobenzenesulfonate,
5-carbamoyl-1H-imidazole-4-yl 2′-nitrobenzenesulfonate,
5-carbamoyl-1H-imidazole-4-yl 4′-methoxybenzenesulfonate, or
5-carbamoyl-1H-imidazole-4-yl p-fluorobenzenesulfonate.

**Revendications pour les Etats contractants: BE CH DE FR GB IT LI NL SE**

1. Composé ou sel non toxique de composé de formule:

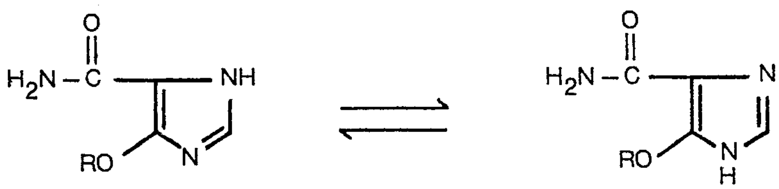

dans laquelle R est un groupe hétéro-aryle ayant un noyau pentagonal ou hexagonal contenant un atome d'azote, de soufre ou d'oxygène qui peut être substitué avec un atome d'halogène, un groupe alkyle en $C_1$ à $C_6$, un groupe aralkyle en $C_7$ ou $C_8$, un groupe alcanoyle en $C_1$ à $C_6$, ou un groupe aroyle qui peut, lui même, être substitué avec un groupe alkyle en $C_1$ à $C_6$, un groupe alkoxy en $C_1$ à $C_6$ ou un atome d'halogène; un groupe alkylsulfonyle en $C_1$ à $C_{18}$; un groupe benzènesulfonyle qui peut être substitué avec un groupe alkyle en $C_1$ à $C_6$, un groupe alkoxy en $C_1$ à $C_6$, un groupe nitro ou un atome d'halogène; ou un groupe cycloalcanoyle à noyau de 3 à 8 chaînons qui peut être substitué avec un groupe alkyle en $C_1$ à $C_6$, un groupe alcényle en $C_2$ à $C_6$, un atome d'halogène, un groupe halogénalcényle en $C_2$ à $C_6$, un groupe phényle, un groupe halogénophényle, un groupe phényle substitué avec un groupe alkyle en $C_1$ à $C_6$ ou un groupe alkoxy en $C_1$ à $C_6$, un groupe formyle, un groupe hydroxy, un groupe amino, un groupe carboxyle, un groupe aminométhyle ou un groupe oxo.

2. Composé suivant la revendication 1, dans lequel R est un groupe 2-furannecarboxyle, 2-thiophènecarbonyle, nicotinoyle, isonicotinoyle, chlorhydrate d'isonicotinoyle ou 3-furannecarboxyle.

3. Composé suivant la revendication 1, dans lequel R est un groupe benzènesulfonyle, *p*-toluènesulfonyl, *p*-chlorobenzènesulfonyle, *o*-nitrobenzènesulfonyle, *p*-méthoxybenzènesulfonyle ou *p*-fluorobenzènesulfonyle.

4. Composé suivant la revendication 1, dans lequel R est le groupe méthanesulfonyle.

5. Composé suivant la revendication 1, dans lequel R est un groupe 1-cyclohexanecarbonyle, 1-cyclopropanecarbonyle ou 1-cyclobutanecarbonyle.

6. Composé suivant la revendication 1, dans lequel R est le groupe N-méthylpyrrole-2-carbonyle ou 5-méthyl-2-thiophènecarbonyle.

7. Composé suivant la revendication 1, dans lequel R est le groupe 2,2-diméthyl-3-(2-méthyl-1-propényl)-1-cyclopropanecarbonyle, 2,2-diméthyl-3-(2,2-dichlorovinyl)-1-cyclopropanecarbonyle, 2,2,3,3-tétraméthyl-1-cyclopropanecarbonyle, 1-(*p*-chlorophényl)-1-cyclopropanecarbonyle, 1-méthyl-1-cyclohexanecarbonyle, 1-méthyl-1-cyclopropanecarbonyle ou 2,2-dichloro-1-méthyl-1-cyclopropanecarbonyle.

8. Le cyclopentane-1'-carboxylate de 5-carbamoyl-1H-imidazole-4-yle,
le cycloheptane-1'-carboxylate de 5-carbamoyl-1H-imidazole-4-yle,
le cyclo-octane-1'-carboxylate de 5-carbamoyl-1H-imidazole-4-yle,
le 1'-(p-méthoxyphényl)cyclopentane-1'-carboxylate de 5-carbamoyl-1H-imidazole-4-yle,
le 1'-phénylcyclopentane-1'-carboxylate de 5-carbamoyl-1H-imidazole-4-yle,
le 1'-(p-tolyl)cyclopentane-1'-carboxylate de 5-carbamoyl-1H-imidazole-4-yle,
le 3'-formylcyclohexane-1'-carboxylate de 5-carbamoyl-1H-imidazole-4-yle,
le 1'-hydroxycyclohexane-1'-carboxylate de 5-carbamoyl-1H-imidazole-4-yle,
le 1'-aminocyclopentane-1'-carboxylate de 5-carbamoyl-1H-imidazole-4-yle,
le 2'-carboxycyclohexane-1'-carboxylate de 5-carbamoyl-1H-imidazole-4-yle,
le 4'-aminométhylcyclohexane-1'-carboxylate de 5-carbamoyl-1H-imidazole-4-yle,
le 3'-oxocyclopentane-1'-carboxylate de 5-carbamoyl-1H-imidazole-4-yle,
le 1'-chloro-2'-oxocyclopentane-1'-carboxylate de 5-carbamoyl-1H-imidazole-4-yle,
le cyclohexane-1'-carboxylate de 5-carbamoyl-1H-imidazole-4-yle,
le cyclopropane-1'-carboxylate de 5-carbamoyl-1H-imidazole-4-yle,
le 2',2'-diméthyl-3'-(2-méthyl-1-propényl)cyclopropane-1'-carboxylate de 5-carbamoyl-1H-imidazole-4-yle,
le 2',2'-diméthyl-3'-(2,2-dichlorovinyl)cyclopropane-1'-carboxylate de 5-carbamoyl-1H-imidazole-4-yle,
le 2',2',3',3'-tétraméthylcyclopropane-1'-carboxylate de 5-carbamoyl-1H-imidazole-4-yle,
le 1'-(p-chlorophényl)cyclopropane-1'-carboxylate de 5-carbamoyl-1H-imidazole-4-yle,
le 1'-méthylcyclohexane-1'-carboxylate de 5-carbamoyl-1H-imidazole-4-yle,
le 1'-méthylcyclopropane-1'-carboxylate de 5-carbamoyl-1H-imidazole-4-yle,
le 2',2'-dichloro-1'-méthylcyclopropane-1'-carboxylate de 5-carbamoyl-1H-imidazole-4-yle ou
le cyclobutane-1'-carboxylate de 5-carbamoyl-1H-imidazole-4-yle.

9. Le picolinate de 5-carbamoyl-1H-imidazole-4-yle,
le N-oxyde du picolinate de 5-carbamoyl-1H-imidazole-4-yle,

le N-oxyde du nicotinate de 5-carbamoyl-1H-imidazole-4-yle,
le N-oxyde de l'isonicotinate du 5-carbamoyl-1H-imidazole-4-yle,
le pyrazine-2'-carboxylate de 5-carbamoyl-1H-imidazole-4-yle,
le 5'-benzylfuranne-3'-carboxylate de 5-carbamoyl-1H-imidazole-4-yle,
le 6'-chloronicotinate de 5-carbamoyl-1H-imidazole-4-yle,
le 2'-chloronicotinate de 5-carbamoyl-1H-imidazole-4-yle,
le 5'-acétylfuranne-2'-carboxylate de 5-carbamoyl-1H-imidazole-4-yle,
le nicotinate de 5-carbamoyl-1H-imidazole-4-yle,
l'isonicotinate de 5-carbamoyl-1H-imidazole-4-yle,
le chlorhydrate de l'isonicotinate de 5-carbamoyl-1H-imidazole-4-yle,
le N-méthylpyrrole-2'-carboxylate de 5-carbamoyl-1H-imidazole-4-yle,
le furanne-2'-carboxylate de 5-carbamoyl-1H-imidazole-4-yle,
le thiophène-2'-carboxylate de 5-carbamoyl-1H-imidazole-4-yle,
le furanne-3'-carboxylate de 5-carbamoyl-1H-imidazole-4-yle,
le 5'-méthylthiophène-2'-carboxylate de 5-carbamoyl-1H-imidazole-4-yle,ou
le thiophène-3'-carboxylate de 5-carbamoyl-1H-imidazole-4-yle.

10. L'éthanesulfonate de 5-carbamoyl-1H-imidazole-4-yle,
le propane-1'-sulfonate de 5-carbamoyl-1H-imidazole-4-yle,
le butane-1'-sulfonate de 5-carbamoyl-1H-imidazole-4-yle,
le pentane-1'-sulfonate de 5-carbamoyl-1H-imidazole-4-yle,
l'hexane-1'-sulfonate de 5-carbamoyl-1H-imidazole-4-yle,
le décane-1'-sulfonate de 5-carbamoyl-1H-imidazole-4-yle,
l'hexadécane-1'-sulfonate de 5-carbamoyl-1H-imidazole-4-yle,
l'octadécane-1'-sulfonate de 5-carbamoyl-1H-imidazole-4-yle, ou
le méthanesulfonate de 5-carbamoyl-1H-imidazole-4-yle.

11. Le benzènesulfonate de 5-carbamoyl-1H-imidazole-4-yle,
le 4'-méthylbenzène sulfonate de 5-carbamoyl-1H-imidazole-4-yle,
le 4'-chlorobenzènesulfonate de 5-carbamoyl-1H-imidazole-4-yle,
le 2'-nitrobenzènesulfonate de 5-carbamoyl-1H-imidazole-4-yle,
le 4'-méthoxybenzènesulfonate de 5-carbamoyl-1H-imidazole-4-yle, ou
le p-fluorobenzènesulfonate de 5-carbamoyl-1H-imidazole-4-yle.

12. Procédé de production d'un composé de formule:

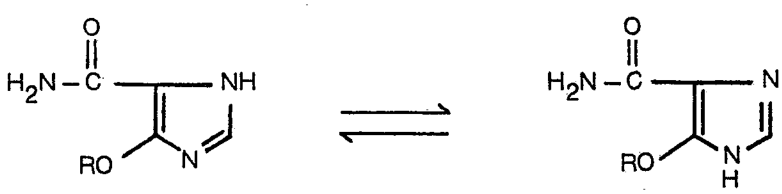

dans laquelle R a la définition donnée dans la revendication 1, qui consiste à faire réagir un 4-carbamoylimidazolium-5-olate de formule:

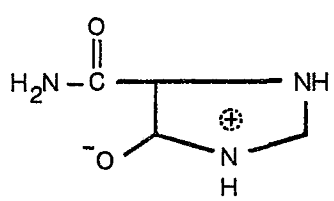

ou un dérivé réactif de ce composé, avec un dérivé réactif d'un acide carboxylique ou d'un acide sulfonique de formule:

R—OH

dans laquelle R a la définition donnée dans la revendication 1.

13. Composition antitumorale ou à effet immunosuppresseur, qui comprend une quantité efficace d'un composé suivant l'une quelconque des revendications 1 à 11 comme ingrédient actif et un support ou diluant pharemaceutiquement acceptable.

**Revendications pour l'Etat contractant: AT**

1. Procédé de production d'un composé de formule:

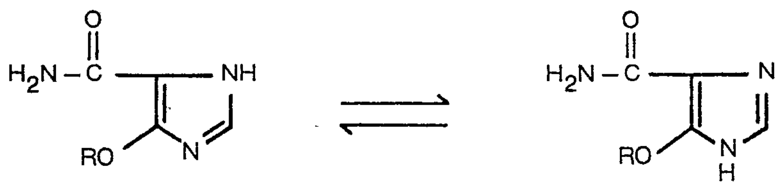

qui consiste à faire réagir un 4-carbamoylimidazolium-5-olate de formule:

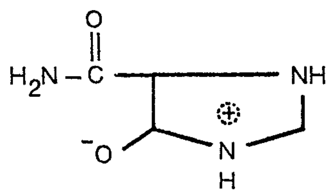

ou un dérivé réactif de ce composé avec un dérivé réactif d'un acide carboxylique ou d'un acide sulfonique de formule:

R—OH

dans laquelle R est un groupe hétéro-aryle ayant un noyau pentagonal ou hexagonal contenant un atome d'azote, de soufre ou d'oxygène qui peut être substitué avec un atome d'halogène, un groupe alkyle en $C_1$ à $C_6$, un groupe aralkyle en $C_7$ ou $C_8$, un groupe alcanoyle en $C_1$ à $C_6$, ou un groupe aroyle qui peut, lui-même, être substitué avec un groupe alkyle en $C_1$ à $C_6$, un groupe alkoxy en $C_1$ à $C_6$ ou un atome d'halogène; un groupe alkylsulfonyle en $C_1$ à $C_{18}$; un groupe benzènesulfonyle qui peut être substitué avec un groupe alkyle en $C_1$ à $C_6$, un groupe alkoxy en $C_1$ à $C_6$, un groupe nitro ou un atome d'halogène; ou un groupe cycloalcanoyle à noyau de 3 à 8 chaînons qui peut être substitué avec un groupe alkyle en $C_1$ à $C_6$, un groupe alcényle en $C_2$ à $C_6$, un atome d'halogène, un groupe halogénalcényle en $C_2$ à $C_6$, un groupe phényle, un groupe halogénophényle, un groupe phényle substitué avec un groupe alkyle en $C_1$ à $C_6$ ou un groupe alkoxy en $C_1$ à $C_6$, un groupe formyle, un groupe hydroxy, un groupe amino, un groupe carboxyle, un groupe aminométhyle ou un groupe oxo.

2. Procedé suivant la revendication 1, dans lequel R est un groupe 2-furannecarboxyle, 2-thiophènecarbonyle, nicotinoyle, isonicotinoyle, chlorhydrate d'isonicotinoyle ou 3-furannecarboxyle.

3. Procedé suivant la revendication 1, dans lequel R est un groupe benzènesulfonyle, *p*-toluènesulfonyle, *p*-chlorobenzènesulfonyle, *o*-nitrobenzènesulfonyle, *p*-méthoxybenzènesulfonyle ou *p*-fluorobenzènesulfonyle.

4. Procedé suivant la revendication 1, dans lequel R est le groupe méthanesulfonyle.

5. Procedé suivant la revendication 1, dans lequel R est un groupe 1-cyclohexanecarbonyle, 1-cyclopropanecarbonyle ou 1-cyclobutanecarbonyle.

6. Procedé suivant la revendication 1, dans lequel R est le groupe N-méthylpyrrole-2-carbonyle ou 5-méthyl-2-thiophènecarbonyle.

7. Procedé suivant la revendication 1, dans lequel R est le groupe 2,2-diméthyl-3-(2-méthyl-1-propényl)-1-cyclopropanecarbonyle, 2,2-diméthyl-3-(2,2-dichlorovinyl)-1-cyclopropanecarbonyle, 2,2,3,3-tétraméthyl-1-cyclopropanecarbonyle, 1-(*p*-chlorophényl)-1-cyclopropanecarbonyle, 1-méthyl-1-cyclohexanecarbonyle, 1-méthyl-1-cyclopropanecarbonyle ou 2,2-dichloro-1-méthyl-1-cyclopropanecarbonyle.

8. Procédé suivant la revendication 1, pour la production des composés suivants:
le cyclopentane-1'-carboxylate de 5-carbamoyl-1H-imidazole-4-yle,
le cycloheptane-1'-carboxylate de 5-carbamoyl-1H-imidazole-4-yle,
le cyclo-octane-1'-carboxylate de 5-carbamoyl-1H-imidazole-4-yle,
le 1'-(p-méthoxyphényl)cyclopentane-1'-carboxylate de 5-carbamoyl-1H-imidazole-4-yle,
le 1'-phénylcyclopentane-1'-carboxylate de 5-carbamoyl-1H-imidazole-4-yle,
le 1'-(p-tolyl)cyclopentane-1'-carboxylate de 5-carbamoyl-1H-imidazole-4-yle,
le 3'-formylcyclohexane-1'-carboxylate de 5-carbamoyl-1H-imidazole-4-yle,
le 1'-hydroxycyclohexane-1'-carboxylate de 5-carbamoyl-1H-imidazole-4-yle,
le 1'-aminocyclopentane-1'-carboxylate de 5-carbamoyl-1H-imidazole-4-yle,
le 2'-carboxycyclohexane-1'-carboxylate de 5-carbamoyl-1H-imidazole-4-yle,
le 4'-aminométhylcyclohexane-1'-carboxylate de 5-carbamoyl-1H-imidazole-4-yle,
le 3'-oxocyclopentane-1'-carboxylate de 5-carbamoyl-1H-imidazole-4-yle,

le 1'-chloro-2'-oxocyclopentane-1'-carboxylate de 5-carbamoyl-1H-imidazole-4-yle,
le cyclohexane-1'-carboxylate de 5-carbamoyl-1H-imidazole-4-yle,
le cyclopropane-1'-carboxylate de 5-carbamoyl-1H-imidazole-4-yle,
le 2',2'-diméthyl-3'-(2-méthyl-1-propényl)cyclopropane-1'-carboxylate de 5-carbamoyl-1H-imidazole-4-yle,
le 2',2'-diméthyl-3'-(2,2-dichlorovinyl)cyclopropane-1'-carboxylate de 5-carbamoyl-1H-imidazole-4-yle,
le 2',2',3',3'-tétraméthylcyclopropane-1'-carboxylate de 5-carbamoyl-1H-imidazole-4-yle,
le 1'-(p-chlorophényl)cyclopropane-1'-carboxylate de 5-carbamoyl-1H-imidazole-4-yle,
le 1'-méthylcyclohexane-1'-carboxylate de 5-carbamoyl-1H-imidazole-4-yle,
le 1'-méthylcyclopropane-1'-carboxylate de 5-carbamoyl-1H-imidazole-4-yle,
le 2',2'-dichloro-1'-méthylcyclopropane-1'-carboxylate de 5-carbamoyl-1H-imidazole-4-yle ou
le cyclobutane-1'-carboxylate de 5-carbamoyl-1H-imidazole-4-yle.

9. Procédé suivant la revendication 1, pour la production des composés suivants:
le picolinate de 5-carbamoyl-1H-imidazole-4-yle,
le N-oxyde du picolinate de 5-carbamoyl-1H-imidazole-4-yle,
le N-oxyde du nicotinate de 5-carbamoyl-1H-imidazole-4-yle,
le N-oxyde de l'isonicotinate du 5-carbamoyl-1H-imidazole-4-yle,
le pyrazine-2'-carboxylate de 5-carbamoyl-1H-imidazole-4-yle,
le 5'-benzylfuranne-3'-carboxylate de 5-carbamoyl-1H-imidazole-4-yle,
le 6'-chloronicotinate de 5-carbamoyl-1H-imidazole-4-yle,
le 2'-chloronicotinate de 5-carbamoyl-1H-imidazole-4-yle,
le 5'-acétylfuranne-2'-carboxylate de 5-carbamoyl-1H-imidazole-4-yle,
le nicotinate de 5-carbamoyl-1H-imidazole-4-yle,
l'isonicotinate de 5-carbamoyl-1H-imidazole-4-yle,
le chlorhydrate de l'isonicotinate de 5-carbamoyl-1H-imidazole-4-yle,
le N-méthylpyrrole-2'-carboxylate de 5-carbamoyl-1H-imidazole-4-yle,
le furanne-2'-carboxylate de 5-carbamoyl-1H-imidazole-4-yle,
le thiophène-2'-carboxylate de 5-carbamoyl-1H-imidazole-4-yle,
le furanne-3'-carboxylate de 5-carbamoyl-1H-imidazole-4-yle,
le 5'-méthylthiophène-2'-carboxylate de 5-carbamoyl-1H-imidazole-4-yle, ou
le thiophène-3'-carboxylate de 5-carbamoyl-1H-imidazole-4-yle.

10. Procédé suivant la revendication 1, pour la production des composés suivants:
l'éthanesulfonate de 5-carbamoyl-1H-imidazole-4-yle,
le propane-1'-sulfonate de 5-carbamoyl-1H-imidazole-4-yle,
le butane-1'-sulfonate de 5-carbamoyl-1H-imidazole-4-yle,
le pentane-1'-sulfonate de 5-carbamoyl-1H-imidazole-4-yle,
l'hexane-1'-sulfonate de 5-carbamoyl-1H-imidazole-4-yle,
le décane-1'-sulfonate de 5-carbamoyl-1H-imidazole-4-yle,

11. Procédé suivant la revendication 1, pour la production des composés suivants:
l'hexadécane-1'-sulfonate de 5-carbamoyl-1H-imidazole-4-yle,
l'octadécane-1'-sulfonate de 5-carbamoyl-1H-imidazole-4-yle, ou
le méthanesulfonate de 5-carbamoyl-1H-imidazole-4-yle.
le benzènesulfonate de 5-carbamoyl-1H-imidazole-4-yle,
le 4'-méthylbenzène sulfonate de 5-carbamoyl-1H-imidazole-4-yle,
le 4'-chlorobenzènesulfonate de 5-carbamoyl-1H-imidazole-4-yle,
le 2'-nitrobenzènesulfonate de 5-carbamoyl-1H-imidazole-4-yle,
le 4'-méthoxybenzènesulfonate de 5-carbamoyl-1H-imidazole-4-yle, ou
le p-fluorobenzènesulfonate de 5-carbamoyl-1H-imidazole-4-yle.

**Patentansprüche für die Vertragsstaaten: BE CH DE FR GB IT LI NL SE**

1. Verbindung der Formel

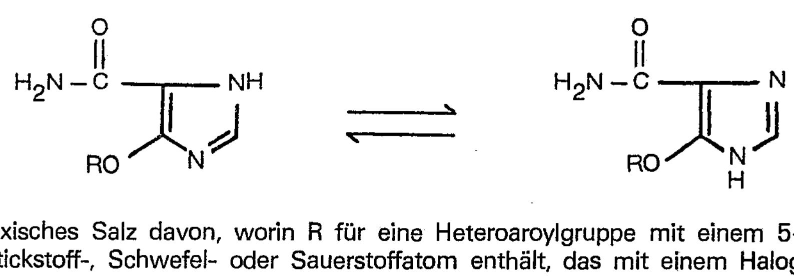

oder ein nichttoxisches Salz davon, worin R für eine Heteroaroylgruppe mit einem 5- oder 6-gliedrigen Ring, der ein Stickstoff-, Schwefel- oder Sauerstoffatom enthält, das mit einem Halogenatom, einer $C_1$- bis $C_6$-Alkylgruppe, einer $C_7$- oder $C_8$-Aralkylgruppe, einer $C_1$- bis $C_6$-Alkanoylgruppe oder einer Aroylgruppe substituiert ist, die selbst mit einer $C_1$- bis $C_6$-Alkylgruppe, einer $C_1$- bis $C_6$-Alkoxygruppe oder

einem Halogenatom substituiert sein kann, eine $C_1$- $C_{18}$-Alkylsulfonylgruppe, eine Benzolsulfonylgruppe, die mit einer $C_1$- bis $C_6$-Alkylgruppe, einer $C_1$—$C_6$-Alkoxygruppe, einer Nitrogruppe oder einem Halogenatom substituiert sein kann, oder eine Cycloalkanoylgruppe mit einem 3- bis 8-gliedrigen Ring steht, der mit einer $C_1$- bis $C_6$-Alkylgruppe, einer $C_2$- bis $C_6$-Alkenylgruppe, einem Halogenatom, einer $C_2$- bis $C_6$-Halogenalkenylgruppe, einer Phenylgruppe, einer Halogenphenylgruppe, einer Phenylgruppe, substituiert mit einer $C_1$- bis $C_6$-Alkylgruppe oder einer $C_1$- bis $C_6$-Alkoxygruppe, einer Formylgruppe, einer Hydroxygruppe, einer Aminogruppe, einer Carboxylgruppe, einer Aminomethylgruppe oder einer Oxogruppe, substituiert sein kann.

2. Verbindung nach anspruch 1, dadurch gekennzeichnet, daß R für 2-Furancarbonyl, 2-Thiophencarbonyl, Nicotinoyl, Isonicotinoyl, Isonicotinoylhydrochlorid oder 3-Furancarbonyl steht.

3. Verbindung nach Anspruch 1, dadurch gekennzeichnet, daß R für Benzolsulfonyl, p-Toluolsulfonyl, p-Chlorbenzolsulfonyl, o-Nitrobenzolsulfonyl, p-Methoxybenzolsulfonyl oder p-Fluorbenzolsulfonyl steht.

4. Verbindung nach Anspruch 1, dadurch gekennzeichnet, daß R für Methansulfonyl steht.

5. Verbindung nach Anspruch 1, dadurch gekennzeichnet, daß R für 1-Cyclohexancarbonyl, 1-Cyclopropancarbonyl oder 1-Cyclobutancarbonyl steht.

6. Verbindung nach Anspruch 1, dadurch gekennzeichnet, daß R für N-methylpyrrol-2-carbonyl oder 5-Methyl-2-thiophencarbonyl steht.

7. Verbindung nach Anspruch 1, dadurch gekennzeichnet, daß R für
2,2-Dimethyl-3-(2-methyl-1-propenyl)-1-cyclopropancarbonyl,
2,2-Dimethyl-3-(2,2-dichlorvinyl)-1-cyclopropancarbonyl,
2,2,3,3-Tetramethyl-1-cyclopropancarbonyl,
1-(p-Chlorphenyl)-1-cyclopropancarbonyl,
1-Methyl-1-cyclohexancarbonyl,
1-Methyl-1-cyclopropancarbonyl oder
2,2-Dichlor-1-methyl-1-cyclopropancarbonyl steht.

8. 5-Carbamoyl-1H-imidazol-4-yl-cyclopentan-1′-carboxylat,
5-Carbamoyl-1H-imidazol-4-yl-cycloheptan-1′-carboxylat,
5-Carbamoyl-1H-imidazol-4-yl-cyclooctan-1′-carboxylat,
5-Carbamoyl-1H-imidazol-4-yl-1′-(p-methoxyphenyl)cyclopentan-1′-carboxylat,
5-Carbamoyl-1H-imidazol-4-yl-1′-phenylcyclopentan-1′-carboxylat,
5-Carbamoyl-1H-imidazol-4-yl-1′-(p-tolyl)cyclopentan-1′-carboxylat,
5-Carbamoyl-1H-imidazol-4-yl-3′-formylcyclohexan-1′-carboxylat,
5-Carbamoyl-1H-imidazol-4-yl-1′-hydroxycyclohexan-1′-carboxylat,
5-Carbamoyl-1H-imidazol-4-yl-1′-aminocyclopentan-1′-carboxylat,
5-Carbamoyl-1H-imidazol-4-yl-2′-carboxycyclohexan-1′-carboxylat,
5-Carbamoyl-1H-imidazol-4-yl-4′-aminomethylcyclohexan-1′-carboxylat,
5-Carbamoyl-1H-imidazol-4-yl-3′-oxocyclopentan-1′-carboxylat,
5-Carbamoyl-1H-imidazol-4-yl-1′-chlor-2′-oxocyclopentan-1′-carboxylat,
5-Carbamoyl-1H-imidazol-4-yl-cyclohexan-1′-carboxylat,
5-Carbamoyl-1H-imidazol-4-yl-cyclopropan-1′-carboxylat,
5-Carbamoyl-1H-imidazol-4-yl-2′,2′-dimethyl-3′-(2-methyl-1-propenyl)-cyclopropan-1′-carboxylat,
5-Carbamoyl-1H-imidazol-4-yl-2′,2′-dimethyl-3′-(2,2-dichlorvinyl)cyclopropan-1′-carboxylat,
5-Carbamoyl-1H-imidazol-4-yl-2′,2′,3′,3′-tetramethylcyclopropan-1′-carboxylat,
5-Carbamoyl-1H-imidazol-4-yl-1′-(p-chlorphenyl)cyclopropan-1′-carboxylat,
5-Carbamoyl-1H-imidazol-4-yl-1′-methylcyclohexan-1′-carboxylat,
5-Carbamoyl-1H-imidazol-4-yl-1′methylcyclohexan-1′-carboxylat
5-Carbamoyl-1H-imidazol-4-yl-1′-methylcyclopropan-1′-carboxylat,
5-Carbamoyl-1H-imidazol-4-yl-2′,2′-dichlor-1′-methylcyclopropan-1′-carboxylat, oder
5-Carbamoyl-1H-imidazol-4-yl-cyclobutan-1′-carboxylat.

9. 5-Carbamoyl-1H-imidazol-4-yl-picolinat,
5-Carbamoyl-1H-imidazol-4-yl-picolinat-N-oxid,
5-Carbamoyl-1H-imidazol-4-yl-nicotinat-N-oxid,
5-Carbamoyl-1H-imidazol-4-yl-isonicotinat-N-oxid,
5-Carbamoyl-1H-imidazol-4-yl-pyrazin-2′-carboxylat,
5-Carbamoyl-1H-imidazol-4-yl-5′-benzylfuran-3′-carboxylat,
5-Carbamoyl-1H-imidazol-4-yl-6′-chlornicotinat,
5-Carbamoyl-1H-imidazol-4-yl-2′-chlornicotinat,
5-Carbamoyl-1H-imidazol-4-yl-5′-acetylfuran-2′-carboxylat,
5-Carbamoyl-1H-imidazol-4-yl-nicotinat,
5-Carbamoyl-1H-imidazol-4-yl-isonicotinat,
5-Carbamoyl-1H-imidazol-4-yl-isonicotinathydrochlorid,
5-Carbamoyl-1H-imidazol-4-yl-N-methylpyrrole-2′-carboxylat,
5-Carbamoyl-1H-imidazol-4-yl-furan-2′-carboxylat,
5-Carbamoyl-1H-imidazol-4-yl-thiophen-2′-carboxylat,

5-Carbamoyl-1H-imidazol-4-yl-furan-3'-carboxylat,
5-Carbamoyl-1H-imidazol-4-yl-5'-methylthiophen-2'-carboxylat, oder
5-Carbamoyl-1H-imidazol-4-yl-thiophen-3'-carboxylat.

10. 5-Carbamoyl-1H-imidazol-4-yl-ethansulfonat,
5-Carbamoyl-1H-imidazol-4-yl-propan-1'-sulfonat,
5-Carbamoyl-1H-imidazol-4-yl-butan-1'-sulfonat,
5-Carbamoyl-1H-imidazol-4-yl-pentan-1'-sulfonat,
5-Carbamoyl-1H-imidazol-4-yl-hexan-1'-sulfonat,
5-Carbamoyl-1H-imidazol-4-yl-decan-1'-sulfonat,
5-Carbamoyl-1H-imidazol-4-yl-hexadecan-1'-sulfonat,
5-Carbamoyl-1H-imidazol-4-yl-octadecan-1'-sulfonat, oder
5-Carbamoyl-1H-imidazol-4-yl-methansulfonat.

11. 5-Carbamoyl-1H-imidazol-4-yl-benzylsulfonat,
5-Carbamoyl-1H-imidazol-4-yl-4'-methylbenzylsulfonat,
5-Carbamoyl-1H-imidazol-4-yl-4'-chlorbenzolsulfonat,
5-Carbamoyl-1H-imidazol-4-yl-2'-nitrobenzolsulfonat,
5-Carbamoyl-1H-imidazol-4-yl-4'-methoxybenzolsulfonat, oder
5-Carbamoyl-1H-imidazol-4-yl-p-fluorbenzolsulfonat.

12. Verfahren zur Herstellung einer Verbindung der Formel

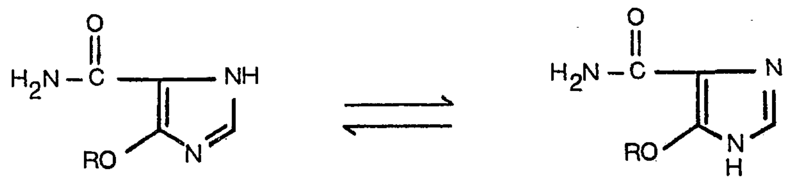

worin R die in Anspruch 1 angegebenen Bedeutungen besitzt, dadurch gekennzeichnet, daß ein 4-Carbamoylimidazolium-5-olat der Formel

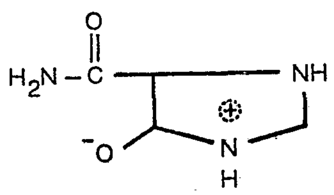

oder ein reaktives Derivat davon mit einem reaktiven Derivat einer Carbonsäure oder Sulfonsäure der Formel

R—OH

worin R die in Anspruch 1 angegebenen Bedeutungen besitzt, zur Umsetzung gebracht wird.

13. Antitumor- oder Antiimmunosuppressanz-Mittel, dadurch gekennzeichnet, daß eine wirksame Menge einer Verbindung gemäß einem der Ansprüche 1 bis 11 als Wirkstoff sowie einen phrmazeutisch verträglichen Träger oder pharmazeutisch verträgliches Verdünnungsmittel enthält.

**Patentansprüche für den Vertragsstaat: AT**

1. Verfahren zur Herstellung einer Verbindung der Formel

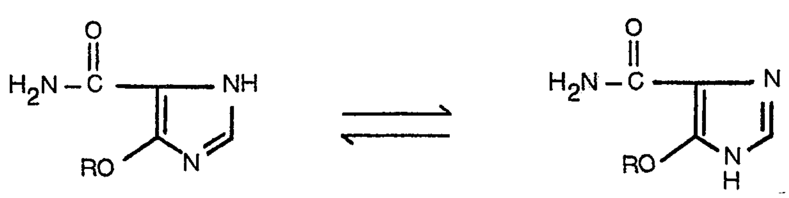

dadurch gekennzeichnet, daß 4-Carbamoylimidazolium-5-olat der Formel

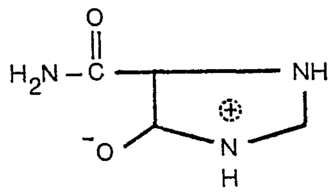

oder ein reaktives Derivat davon mit einem reaktiven Derivat einer Carbonsäure oder einer Sulfonsäure der Formel

R—OH

umgesetzt wird, wobei R eine Heteroaroylgruppe mit einem 5- oder 6-gliedrigen Ring, der ein Stickstoff-, Schwefel- oder Sauerstoffatom enthält, das mit einem Halogenatom, einer $C_1$- bis $C_6$-Alkylgruppe, einer $C_7$- oder $C_8$-Aralkylgruppe, einer $C_1$- bis $C_6$-Alkanoylgruppe oder einer Aroylgruppe substituiert sein kann, die selbst mit einer $C_1$- bis $C_6$-Alkylgruppe, einer $C_1$- bis $C_6$-Alkoxygruppe oder einem Halogenatom substituiert sein kann, oder eine Cycloalkanoylgruppe mit einem 3- bis 8-gruppe, die mit einer $C_1$- bis $C_6$-Alkylgruppe, einer $C_1$- bis $C_6$-Alkoxygruppe, einer Nitrogruppe oder einem Halogenatom substituierten sein kann, oder eine Cycloalkanoylgruppe mit einem 3- bis 8-gliedrigen Ring ist, der mit einer $C_1$- bis $C_6$-Alkylgruppe, einer $C_2$—$C_6$-Alkenylgruppe, einem Halogenatom, einer $C_2$- bis $C_6$-Halogenalkenylgruppe, einer Phenylgruppe, einer Halogenphenylgruppe, einer Phenylgruppe substituiert mit einer $C_1$- bis $C_6$-Alkyl- oder $C_1$- bis $C_6$-Alkoxygruppe, einer Formylgruppe, einer Hydroxygruppe, einer Aminogruppe, einer Carboxylgruppe, einer Aminomethylgruppe oder einer Oxogruppe, substituiert sein kann.

2. Verfahren nach Anspruch 1, dadurch gekennzeichnet, daß R für 2-Furancarbonyl, 2-Thiophencarbonyl, Nicotinoyl, Isonicotinoyl, Isonicotinoylhydrochlorid oder 3-Furancarbonyl steht.

3. Verfahren nach Anspruch 1, dadurch gekennzeichnet, daß R für Benzolsulfonyl, p-Toluolsulfonyl, p-Chlorbenzolsulfonyl, o-nitrobenzolsulfonyl, p-methoxybenzolsulfonyl oder p-Fluorbenzolsulfonyl steht.

4. Verfahren nach Anspruch 1, dadurch gekennzeichnet, daß R für Methansulfonyl steht.

5. Verfahren nach Anspruch 1, dadurch gekennzeichnet, daß R für 1-Cyclohexancarbonyl, 1-Cyclopropancarbonyl oder 1-Cyclobutancarbonyl steht.

6. Verfahren nach Anspruch 1, dadurch gekennzeichnet, daß R für N-Methylpyrrol-2-carbonyl oder 5-Methyl-2-thiophencarbonyl steht.

7. Verfahren nach Anspruch 1, dadurch gekennzeichnet, daß R für 2,2-Dimethyl-3-(2-methyl-1-propenyl)-1-cyclopropancarbonyl, 2,2-Dimethyl-3-(2,2-dichlorvinyl)-1-cyclopropancarbonyl, 2,2,3,3-Tetramethyl-1-cyclopropancarbonyl, 1-(p-chlorphenyl)-1-cyclopropancarbonyl, 1-Methyl-1-cyclohexancarbonyl, 1-Methyl-1-cyclopropancarbonyl oder 2,2-Dichlor-1-methyl-1-cyclopropancarbonyl steht.

8. Verfahren nach Anspruch 1, dadurch gekennzeichnet, daß
5-Carbamoyl-1H-imidazol-4-yl-cyclopentan-1′-carboxylat,
5-Carbamoyl-1H-imidazol-4-yl-cycloheptan-1′-carboxylat,
5-Carbamoyl-1H-imidazol-4-yl-cyclooctan-1′-carboxylat,
5-Carbamoyl-1H-imidazol-4-yl-1′-(p-methoxyphenyl)cyclopentan-1′-carboxylat,
5-Carbamoyl-1H-imidazol-4-yl-1′-phenylcyclopentan-1′-carboxylat,
5-Carbamoyl-1H-imidazol-4-yl-1′-(p-tolyl)cyclopentan-1′-carboxylat,
5-Carbamoyl-1H-imidazol-4-yl-3′-formylcyclohexan-1′-carboxylat,
5-Carbamoyl-1H-imidazol-4-yl-1′-hydroxycyclohexan-1′-carboxylat,
5-Carbamoyl-1H-imidazol-4-yl-1′-aminocyclopentan-1′-carboxylat,
5-Carbamoyl-1H-imidazol-4-yl-2′-carboxycyclohexan-1′-carboxylat,
5-Carbamoyl-1H-imidazol-4-yl-4′-aminomethylcyclohexan-1′-carboxylat,
5-Carbamoyl-1H-imidazol-4-yl-3′-oxocyclopentan-1′-carboxylat,
5-Carbamoyl-1H-imidazol-4-yl-1′-chloro-2′-oxocyclopentan-1′-carboxylat,
5-Carbamoyl-1H-imidazol-4-yl-cyclohexan-1′-carboxylat,
5-Carbamoyl-1H-imidazol-4-yl-cyclopropan-1′-carboxylat,
5-Carbamoyl-1H-imidazol-4-yl-2′,2′-dimethyl-3′-(2-methyl-1-propenyl)-cyclopropan-1′-carboxylat,
5-Carbamoyl-1H-imidazol-4-yl-2′,2′-dimethyl-3′-(2,2-dichlorvinyl)cyclopropan-1′-carboxylat,
5-Carbamoyl-1H-imidazol-4-yl-2′,2′,3′,3′-tetramethylcyclopropan-1′-carboxylat,
5-Carbamoyl-1H-imidazol-4-yl-1′-(p-chlorophenyl)cyclopropan-1′-carboxylat,
5-Carbamoyl-1H-imidazol-4-yl-1′-methylcyclohexan-1′-carboxylat,
5-Carbamoyl-1H-imidazol-4-yl-1′-methylcyclopropan-1′-carboxylat,
5-Carbamoyl-1H-imidazol-4-yl-2′,2′-dichloro-1′-methylcyclopropan-1′-carboxylat, oder
5-Carbamoyl-1H-imidazol-4-yl-cyclobutan-1′-carboxylat hergestellt werden.

9. Verfahren nach Anspruch 1, dadurch gekennzeichnet, daß

5-Carbamoyl-1H-imidazol-4-yl-picolinat,
5-Carbamoyl-1H-imidazol-4-yl-picolinat-N-oxid,
5-Carbamoyl-1H-imidazol-4-yl-nicotinat-N-oxid,
5-Carbamoyl-1H-imidazol-4-yl-isonicotinat-N-oxid,
5-Carbamoyl-1H-imidazol-4-yl-pyrazin-2′-carboxylat,
5-Carbamoyl-1H-imidazol-4-yl-5′-benzylfuran-3′-carboxylat,
5-Carbamoyl-1H-imidazol-4-yl-6′-chloronicotinat,
5-Carbamoyl-1H-imidazol-4-yl-2′-chloronicotinat,
5-Carbamoyl-1H-imidazol-4-yl-5′-acetylfuran-2′-carboxylat,
5-Carbamoyl-1H-imidazol-4-yl-nicotinat,
5-Carbamoyl-1H-imidazol-4-yl-isonicotinat,
5-Carbamoyl-1H-imidazol-4-yl-isonicotinathydrochlorid,
5-Carbamoyl-1H-imidazol-4-yl-N-methylpyrrole-2′-carboxylat,
5-Carbamoyl-1H-imidazol-4-yl-furan-2′-carboxylat,
5-Carbamoyl-1H-imidazol-4-yl-thiophen-2′-carboxylat,
5-Carbamoyl-1H-imidazol-4-yl-furan-3′-carboxylat,
5-Carbamoyl-1H-imidazol-4-yl-5′-methylthiophen-2′-carboxylat, oder
5-Carbamoyl-1H-imidazol-4-yl-thiophen-3′-carboxylat hergestellt werden.

10. Verfahren nach Anspruch 1, dadurchgekennzeichnet, daß
5-Carbamoyl-1H-imidazol-4-yl-ethansulfonat,
5-Carbamoyl-1H-imidazol-4-yl-propan-1′-sulfonat,
5-Carbamoyl-1H-imidazol-4-yl-butan-1′-sulfonat,
5-Carbamoyl-1H-imidazol-4-yl-pentan-1′-sulfonat,
5-Carbamoyl-1H-imidazol-4-yl-hexan-1′-sulfonat,
5-Carbamoyl-1H-imidazol-4-yl-decan-1′-sulfonat hergestellt werden.

11. Verfahren nach Anspruch 1, dadurch gekennzeichnet, daß
5-Carbamoyl-1H-imidazol-4-yl-hexadecan-1′-sulfonat,
5-Carbamoyl-1H-imidazol-4-yl-octadecan-1′-sulfonat, oder
5-Carbamoyl-1H-imidazol-4-yl-methansulfonat
5-Carbamoyl-1H-imidazol-4-yl-benzolsulfonat,
5-Carbamoyl-1H-imidazol-4-yl-4′-methylbenzylsulfonat,
5-Carbamoyl-1H-imidazol-4-yl-4′-chlorbenzolsulfonat,
5-Carbamoyl-1H-imidazol-4-yl-2′-nitrobenzolsulfonat,
5-Carbamoyl-1H-imidazol-4-yl-4′-methoxybenzolsulfonat, oder
5-Carbamoyl-1H-imidazol-4-yl-p-fluorobenzolsulfonat hergestellt werden.